# EUROPÄISCHE PATENTANMELDUNG

(11) **EP 3 287 914 A1**
(43) Veröffentlichungstag der Anmeldung: **28.02.2018**
(21) Anmeldenummer: 16185297.5
(22) Anmeldetag: 23.08.2016
(51) Int. Cl.: G06F 19/00, A61B 5/00

(54) **ERMITTLUNG VON ERGEBNISDATEN AUF BASIS VON MEDIZINISCHEN MESSDATEN AUS VERSCHIEDENEN MESSUNGEN**

(71) Anmelder: Siemens Healthcare GmbH, 91052 Erlangen (DE)
(72) Erfinder: Grimm, Robert, 90459 Nürnberg (DE); Schweizer, Bernd, 68775 Ketsch (DE)

(57) **Zusammenfassung**

Die vorliegende Erfindung betrifft ein Verfahren zur Ermittlung von Ergebnisdaten (ED) auf Basis von medizinischen Messdaten (MD1, MD2, MD3, MD4, MD5) eines Untersuchungsobjekts (O), welche in verschiedenen Messungen (Ia, Ib, Id, Ic, Ie), vorzugsweise mit unterschiedlichen Messgeräten (1, 2, 3), erfasst wurden. Im Rahmen des Verfahren wird ein hochdimensionaler erster Parameterraum (PR, PR1) gebildet, in welchem Messwerte der verschiedenen Messungen (Ia, Ib, Id, Ic, Ie) mit Hilfe von Wertetupeln (TP1) repräsentiert sind, wobei die Messwerte der verschiedener Messungen (Ia, Ib, Id, Ic, Ie) anhand ihrer räumlichen Anordnung im Untersuchungsobjekt (O) und/oder anhand ihrer zeitlichen Anordnung zueinander einem Wertetupel (TP1) zugeordnet sind. Im ersten Parameterraum (PR1) werden die Wertetupel (TP1) unter Nutzung zumindest einer Abbildungsfunktion (AF1) auf zumindest einen weiteren Parameterraum (PR2), welcher eine niedrigere Dimension als der erste Parameterraum (PR1) aufweist, zum Erhalt von Ergebnisdaten (ED) analysiert. Weiterhin erfolgt eine Ausgabe, vorzugsweise Visualisierung, der Ergebnisdaten (ED). Außerdem wird eine entsprechende Einrichtung (10) zur Ermittlung von Ergebnisdaten (ED) beschrieben.

## Beschreibung

Die Erfindung betrifft ein Verfahren zur Ermittlung von Ergebnisdaten auf Basis von medizinischen Messdaten eines Untersuchungsobjekts, welche in verschiedenen Messungen, vorzugsweise mit unterschiedlichen Messgeräten, erfasst wurden. Darüber hinaus betrifft die Erfindung eine entsprechende Einrichtung zur Durchführung dieses Verfahrens.

In der medizinischen Diagnostik, insbesondere der bildgebenden Diagnostik, nimmt die Möglichkeit der multiparametrischen - insbesondere "multimodalen" - Datenaufnahme immer breiteren Raum ein. Unter "Datenaufnahme" ist dabei die Erfassung von medizinischen Messdaten zu verstehen, wobei es sich im Folgenden meist, aber nicht nur, um Bilddaten handelt. Multiparametrisch sind Messdaten hinsichtlich unterschiedlicher Parameter. "Multimodal" heißt dabei, dass die Daten mit unterschiedlichen Modalitäten, d. h. medizintechnischen Messgeräten, insbesondere medizintechnischen bildgebenden Geräten wie MRT (Magnetresonanztomograph), CT (Computertomograph), PET-Geräten (Positronen-Emissions-Tomograph), Ultraschallsystemen etc. akquiriert werden. Beispiele hierfür sind multiparametrische Magnetresonanzprotokolle, z. B. zusätzlich in Verbindung mit einer PET-Aufnahme. An dieser Stelle sei erwähnt, dass multimodale Messdaten in diesem Sinne also auch Messdaten sind, die auf Kombinations-Geräten, wie einem MRT-PET-Gerät, erzeugt wurden, d. h. dass die mit unterschiedlichen Messprinzipien in einem Kombinations-Gerät erfassten Messdaten als Messdaten von unterschiedlichen Modalitäten zu sehen sind.

Traditionell wird die Befundung von multiparametrischen, insbesondere multimodalen, Messdaten bzw. Bildern mit Hilfe verschiedener Ansätze durchgeführt: Bei einer Methode erfolgt eine sequenzielle Darstellung der verschiedenen Kontraste bzw. Bilder, d. h. der Befunder liest die Bilder nacheinander. Ein Beispiel hierfür ist die Erkennung eines Tumors als Hyperintens im hohen b-Wert-Bild einer Diffusionsbildgebung und Hypointens im ADC-Bild der Diffusionsbildgebung (ADC = Apparent Diffusion Coefficient; scheinbarer Diffusionskoeffizient). In einer weiteren Methode werden die Kontraste fusioniert dargestellt. Z. B. kann ein T2-Kontrast einer MRT-Messung mit der PET-Aufnahme überlagert werden, wobei die Bilder hierzu zuvor aufeinander registriert wurden. Insbesondere ist somit die Möglichkeit der Visualisierung und Analyse bisher auf in der Regel eindimensionale Histogramme und zweidimensionale Scatter-Plots beschränkt. Für ganz spezielle Kontrast-Kombinationen bzw. Kombinationen verschiedener Bilder (im Folgenden auch als "Parameterkarten" bezeichnet) sind bereits diagnostische Parameterkombinationen bekannt, wie beispielsweise für den hohen b-Wert und den ADC-Wert in Tumorbefundungen. Dadurch ist es möglich, die Werte vorher rechnerisch zu kombinieren und dann den Kombinationswert in Form einer Parameterkarte darzustellen bzw. auszuwerten. Allerdings sind solche Zusammenhänge für die stetig wachsende Anzahl von möglichen Kontrasten im Allgemeinen nicht bekannt. Solche Vorgehensweisen sind also auf ganz bestimmte Kombinationen von Werten beschränkt, im Prinzip auf solche Fälle, bei denen man bereits vorab weiß, dass bestimmte Wertekombinationen für bestimmte Befundungen relevant sind.

In der klinischen Realität besteht somit die Gefahr, dass die steigende Anzahl von verfügbaren Patientendaten mit traditionellen Befundungsmethoden nicht mehr adäquat ausgewertet werden kann. Dadurch werden die potentiellen Vorteile der multiparametrischen Bildgebung möglicherweise nicht immer optimal ausgenutzt.

Es ist eine Aufgabe der vorliegenden Erfindung, ein alternatives Verfahren zur Ermittlung von Ergebniswerten auf Basis von medizinischen Messdaten eines Untersuchungsobjekts, insbesondere einer Vielzahl von verschiedenen Messungen, sowie eine entsprechende Einrichtung hierfür zu schaffen, welche die Berücksichtigung einer Vielzahl von Messdaten bei einer Befundung erleichtern kann.

Diese Aufgabe wird durch ein Verfahren gemäß Patentanspruch 1 sowie durch eine Einrichtung gemäß Patentanspruch 16 gelöst.

Das erfindungsgemäße Verfahren umfasst zumindest folgende Verfahrensschritte:
Es wird zunächst ein hochdimensionaler erster Parameterraum gebildet, in welchem Messwerte der verschiedenen Messungen mit Hilfe von Wertetupeln repräsentiert sind. Dabei sind die Messwerte der verschiedenen Messungen anhand ihrer räumlichen Anordnung, z. B. bildpunktweise (d. h. voxel- oder pixelweise) oder regionenweise, und/oder anhand ihrer zeitlichen Anordnung zueinander einem Wertetupel zugeordnet. Prinzipiell und meist bevorzugt kann dabei also für jeden Bildpunkt ein einzelner Wertetupel ermittelt werden. Grundsätzlich ist es aber auch möglich, einen gemeinsamen Wertetupel für eine Gruppe von Bildpunkten, insbesondere auch für eine vordefinierte interessierende Region im Untersuchungsobjekt zu ermitteln.

Die Messwerte, die in der Auswertung berücksichtigt werden sollen, werden dabei also als Vektoren in dem hochdimensionalen Parameterraum interpretiert, wobei die Vektoren durch die Wertetupel definiert sind. Die einzelnen Werte des Wertetupels sind dabei die Messwerte hinsichtlich eines bestimmten Messparameters, und die Messparameter bilden somit die Koordinaten des hochdimensionalen Parameterraums. Ein solcher hochdimensionaler Parameterraum weist bevorzugt mindestens vier Dimensionen, vorzugsweise mindestens fünf Dimensionen, besonders bevorzugt aber noch mehr Dimensionen auf, z. B. ganz besonders bevorzugt mindestens zehn Dimensionen oder mindestens zwanzig Dimensionen. Jede Dimension entspricht dabei einem zu berücksichtigenden Parameter.

Nachdem die Messwerte in Form der Wertetupel bzw. Vektoren im (wie zuvor erläutert definierten) hochdimensionalen ersten Parameterraum angeordnet sind, erfolgt innerhalb dieses ersten Parameterraums eine Analyse der Wertetupel unter Nutzung zumindest einer Abbildungsfunktion auf zumindest einen weiteren Parameterraum, welcher eine niedrigere Dimension als der erste Parameterraum aufweist. Diese Analyse umfasst z. B. die Analyse hinsichtlich der absoluten und/oder relativen Lage der Wertetupel, ihre Dichte im Raum etc. Die Abbildungsfunktion kann dabei unterschiedliche Funktionstypen umfassen, die später noch genauer erläutert werden. Durch diese Abbildungsfunktion auf den zweiten Parameterraum wird die Dimension der Wertetupel bzw. Vektoren reduziert. Die dimensionsreduzierten Wertetupel können dann direkt die Endergebnisdaten sein oder, wie später noch erläutert wird, mit weiteren Abbildungsfunktionen noch mehr in ihrer Dimension reduziert werden.

Letztendlich erfolgt dann eine Ausgabe, vorzugsweise eine Visualisierung der (so kombinierten) Ergebnisdaten, also beispielsweise der Vektoren bzw. Wertetupel des niedriger dimensionierten Raums. Vorzugsweise erfolgt im Gesamtergebnis selbst bei einem sehr hochdimensionalen ersten Parameterraum letztlich eine Reduzierung auf maximal vier Dimensionen, besonders bevorzugt auf maximal drei Dimensionen, da sich drei Dimensionen noch grafisch relativ gut darstellen lassen, wobei als vierte Dimension beispielsweise noch eine farbliche oder anderweitige Markierung im dreidimensionalen Raum genutzt werden könnte. Das heißt, es erfolgt bevorzugt eine Reduzierung der Dimensionen so weit, dass die Ergebnisdaten so visualisiert werden können, dass sie vom Befunder leicht erfassbar sind. Wenn der erste Parameterraum selbst nur vier Dimensionen aufweist (was außer bei den z. B. später gegebenen, stark vereinfachten Beispielen eher selten der Fall ist), sollten die Ergebnisdaten zumindest auf drei - vorzugsweise maximal zwei - Dimensionen reduziert sein.

Eine Kernidee innerhalb des Verfahrens besteht also darin, die multiparametrischen medizinischen Daten als hochdimensionalen Parameterraum zu interpretieren. Wie erwähnt, sind diese Messdaten bevorzugt multi-parametrische Bilddaten. Es kann sich aber auch zusätzlich um Labormesswerte, Anamnesedaten oder Daten aus vorherigen Untersuchungszeitpunkten handeln. Außerdem können aus Bilddaten durch geeignete Vorverarbeitungsschritte, wie z. B. Volumetrie durch automatische Organsegmentierung, weitere Parameter abgeleitet werden, die wiederum als zusätzliche Dimensionen interpretiert werden können. Wie später noch gezeigt wird, ermöglicht die Interpretation der verschiedenen Messdaten als zusammenhängende Wertetupel in einem hochdimensionalen Parameterraum eine Vielzahl von Auswertungsmöglichkeiten, die insbesondere auch zumindest teilweise automatisch durchgeführt werden können, z. T. auch vollautomatisch, was die Befundung erheblich unterstützen kann. Die Befundung ist somit nicht mehr darauf beschränkt, dass vorab bestimmte darstellbare Parameterkarten erstellt werden, die dann überlagert werden oder vom Befunder sequenziell betrachtet werden müssen, was die Qualität der Befundung stark von der Erfahrung und dem Wissen des Befunders abhängig macht.

Eine erfindungsgemäße Einrichtung zur Ermittlung von solchen kombinierten Ergebnissen auf Basis von medizinischen Messdaten eines Untersuchungsobjekts aus verschiedenen Messungen ist entsprechend zur Ausführung der oben beschriebenen Verfahrensschritte ausgebildet.

Die wesentlichen Komponenten der erfindungsgemäßen Einrichtung können dabei in Form von Softwarekomponenten ausgebildet sein. Grundsätzlich können diese Komponenten aber auch zum Teil, insbesondere, wenn es um besonders schnelle Berechnungen geht, in Form von softwareunterstützter Hardware, beispielsweise FPGAs oder dergleichen, realisiert sein. Ebenso können die benötigten Schnittstellen, beispielsweise, wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Die erfindungsgemäße Einrichtung kann z. B. mittels der geeigneten Softwarekomponenten auf einer Rechnereinheit realisiert sein, die an sich unabhängig von den Modalitäten bzw. Geräten zur Aufnahme der Messdaten ist, beispielsweise einer Workstation, die die erforderlichen Messdaten, insbesondere Bilddaten, z. B. über ein medizinisches Datennetzwerk wie ein radiologisches Informationssystem (RIS) in einer Praxis oder Klinik von den jeweiligen Modalitäten bzw. Geräten und/oder aus einem Speicher, in dem die Daten hinterlegt sind, übernimmt. Prinzipiell kann die Einrichtung aber auch Teil einer Modalität sein, insbesondere eines Kombinationsgeräts wie eines MRT-PET-Geräts, beispielsweise in dessen Steuereinrichtung oder einer direkt daran angeschlossenen Auswertestation angeordnet sein, um sofort nach der Messung das erfindungsgemäße Verfahren oder Teile davon durchzuführen. Dementsprechend kann die Erfassung der Messdaten zum einen einfach eine Übernahme der entsprechenden fertigen Messdaten, wie Bilddaten bzw. Parameterkarten, umfassen, aber auch Verfahrensschritte der Akquise der Rohdaten und gegebenenfalls eine Rekonstruktion bzw. Berechnung von Bilddaten bzw. Parameterkarten umfassen.

Eine weitgehend softwaremäßige Realisierung hat den Vorteil, dass auch schon bisher verwendete Workstations oder Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Rechnereinheit (beispielsweise einer Workstation eines medizinischen Datennetzwerks) ladbar ist, mit Programmabschnitten, um alle Schritte des erfindungsgemäßen Verfahrens auszuführen, wenn das Programm in der Rechnereinheit ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z. B. Hardware-Schlüssel (Dongles etc.) zur Nutzung der Software, umfassen.

Zum Transport zur Rechnereinheit und/oder zur Speicherung an oder in der Rechnereinheit kann ein computerlesbares Medium, beispielsweise ein Memorystick, eine Festplatte oder ein sonstiger transportabler oder fest eingebauter Datenträger dienen, auf welchem die von der Rechnereinheit einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen einer anderen Anspruchskategorie weitergebildet sein können und die Merkmale verschiedener Ausführungsbeispiele zu neuen Ausführungsbeispielen kombiniert werden können.

Wie bereits oben erwähnt, können die verschiedenen Messungen insbesondere von unterschiedlichen Messgeräten bzw. Modalitäten stammen. Grundsätzlich ist es bei einer bevorzugten Vorgehensweise aber auch möglich, dass die verschiedenen Messungen desselben Objekts bzw. desselben interessierenden Bereichs (ROI, Region of Interest) gleichartige Messungen, also insbesondere Messungen der gleichen Art bzw. des gleichen Typs mit denselben oder annähern gleichen Messparametern umfassen, die lediglich zu verschiedenen Zeitpunkten aufgenommen worden sind. Auch hierbei handelt es sich um verschiedene Messungen. Ein Beispiel hierfür wäre die Messung von Kontrastmittel in einem bestimmten Objekt, wie einem Organ oder Tumorgewebe, zu verschiedenen Zeitpunkten, beispielsweise in angemessenen zeitlichen Abständen aufeinanderfolgend, um so die An- und/oder Abreicherung von Kontrastmittel zu beobachten. Die Messwerte an den verschiedenen Bildpunkten, welche die Kontrastmittelintensität repräsentieren, können dann wiederum als Vektor bzw. Wertetupel interpretiert werden. Hierbei ist auch eine Kombination mit anderen verschiedenen Messungen möglich, d. h. dass beispielsweise dieser Vektor, welcher die zeitlichen Messungen an einem bestimmten Bildpunkt umfasst, nur einen Teil-Vektor bzw. Teil-Wertetupel innerhalb eines Gesamt-Wertetupels bildet. So könnten beispielsweise unter der Kontrastmittelan- und -abreicherung verschiedene Messungen auch mit unterschiedlichen Geräten durchgeführt werden. Ein Wertetupel ist dann beispielsweise einem bestimmtem Bildpunkt zugeordnet und die einzelnen Werte des Wertetupels umfassen einen Teil-Wertetupel, welcher eine erste Messung zu bestimmten Zeiten umfasst, und einen zweiten Teil-Wertetupel, welcher eine andere Messung zu bestimmten Zeitpunkten umfasst, wobei diese Teil-Wertetupel einfach unter Bildung des Gesamt-Wertetupels hintereinander verkettet werden. Werden beispielsweise zwei Parameterwerte jeweils zu zehn Zeitpunkten erfasst, käme so ein Wertetupel zustande, welcher insgesamt zwanzig Werte umfasst. Dieser Wertetupel kann dann als Vektor in einem zwanzigdimensionalen Parameterraum interpretiert und im Rahmen der Erfindung entsprechend analysiert werden.

Wie bereits oben erwähnt, gibt es verschiedene Möglichkeiten von Abbildungsfunktionen. Zu den besonders bevorzugten Funktionstypen gehören dabei:
- Eine Abbildung in einen Farbraum oder einen Raum, welcher einen Farbraum (als Unterraum) umfasst, beispielsweise einen RGB-Farbraum, bei dem drei Dimensionen durch die Farben Rot (R), Grün (G) und Blau (B) definiert sind.
- Eine Abbildung bzw. Zuweisung in diskrete Klassen. Solche diskreten Klassen könnten z. B. Klassifizierungen wie das System mit den Klassen PI-RADS 1 bis 5 bei Prostatakarzinomen sein. Das heißt, bei einer solchen Abbildungsfunktion kann also eine - zumindest teilweise automatische - Klassifizierung des Untersuchungsobjekts und/oder von Teilobjekten, z. B. von bestimmten Regionen, erfolgen. Dies ist, wie später noch anhand von Beispielen gezeigt wird, vorzugsweise auf Basis der Anordnung der Wertetupel in einem der Parameterräume möglich.
- Eine Abbildung in einen Raum mit klinisch relevanten semantischen Koordinatenachsen bzw. Dimensionen, wie beispielsweise benign/malign. Wenn beispielsweise bekannt ist, dass eine bestimmte Parameterkombination mit sehr hoher Wahrscheinlichkeit auf Malignität schließen lässt, während eine andere Parameterkombination charakteristisch für benignes Gewebe ist, lässt sich ein neu zu klassifizierendes Gewebe anhand des Abstandes zu diesen Referenzpunkten eine Wahrscheinlichkeit für entsprechende Klassenzugehörigkeit angeben.;
- Eine Abbildung in ein Koordinatensystem mit mathematischen Vorzugseigenschaften. Ein Beispiel hierfür wäre die noch später näher erläuterte Nutzung einer Hauptachsentransformation.
- Eine Integration und/oder Projektion über (vor-)definierte Bereiche des Parameterraums. Ein Beispiel hierfür wäre die Summation von Voxeln, die in einem bestimmten Parameterbereich liegen, um so das Volumen oder die Gesamt- "Last" zu evaluieren. Würde der bestimmte Parameterbereich tumoröses Gewebe unterschiedlichen Grades charakterisieren, kann z. B. durch die Summation die Gesamt-Belastung durch tumoröses Gewebe bestimmt werden. Dabei kann auch ein hohes Volumen mit niedrigem Tumorgrad zur selben Gesamt-Last führen wie niedriges Volumen mit hochgradigem Tumorgewebe. Ein weiteres Beispiel ist die Prozessierung von Daten mit einer temporalen Dimension. Hier kann die Summation benutzt werden, um die Fläche unterhalb der Zeitkurve bzw. die zeitliche Variabilität jedes Voxels abzubilden, oder eine Projektion, um beispielsweise das temporale Minimum oder Maximum des Parameters zu bestimmen.

Ebenso ist eine Kombination der obigen Funktionstypen und/oder weiterer Funktionstypen möglich, um Abbildungsfunktionen zu bilden, die es erlauben, von einem hochdimensionalen Parameterraum auf einen niedriger dimensionierten Parameterraum abzubilden.

Bei einer ganz besonders bevorzugten Vorgehensweise erfolgt die Dimensionsreduzierung mehrstufig. Hierzu können vorzugsweise zum Erhalt der Ergebnisdaten zunächst durch eine erste Abbildungsfunktion vom ersten Parameterraum auf einen zweiten Parameterraum Interims-Ergebnisdaten ermittelt werden. Diese Interims-Ergebnisdaten können dann z. B. wieder in Form von Wertetupeln im zweiten Parameterraum, d. h. dem niedriger dimensionierten Parameterraum, vorliegen. Die Interims-Ergebnisdaten im zweiten Parameterraum können dann wiederum analysiert werden, wobei die Analyse wieder unter Nutzung zumindest einer zweiten Abbildungsfunktion auf zumindest einen dritten Parameterraum erfolgt, der dann eine niedrigere Dimension als der zweite Parameterraum aufweist. Hierbei ergibt sich u. a. der Vorteil, dass in jedem Parameterraum eine geeignete Analyse für die dort vorliegenden Parameterwerte durchgeführt werden kann.

Besonders bevorzugt umfassen die erste Abbildungsfunktion und die zweite Abbildungsfunktion dabei unterschiedliche Funktionstypen bzw. sie beruhen auf unterschiedlichen Analyseprinzipien. Zum Beispiel wäre es möglich, dass in einer ersten Abbildungsfunktion zunächst eine später noch genauer beschriebene Gruppierung bzw. Clusterung von Wertetupeln erfolgt und weiter anhand dieser Cluster eine Hauptachsenanalyse, um so zunächst die Dimension des hochdimensionalen ersten Parameterraums um eine oder mehrere Dimensionen zu reduzieren. In dem zweiten Abbildungsraum, welcher Koordinatenachsen aufweisen kann, die durch die Hauptachsen gegeben sein können, können dann wiederum Trennlinien oder dergleichen gesetzt werden, um so eine weitere Dimensionsreduzierung zu erreichen und bestimmte Wertetupel bestimmten Bereichen oder Charakteristika zuzuordnen, was, wie oben erläutert, einer weiteren Abbildungsfunktion entspricht.

Für die Analyse der Wertetupel in einem Parameterraum gibt es folglich unterschiedliche Möglichkeiten. Im Folgenden werden einige besonders wesentliche Varianten genannt, wobei auch jederzeit eine Kombination der unterschiedlichen Varianten möglich ist.

Bevorzugt erfolgt die Analyse, insbesondere auch eine Zuordnung zu Klassen oder dergleichen, unter Berücksichtigung von Positionen der Wertetupel in einem Parameterraum in Bezug zu einer Grenz-Hyperebene des Parameterraums. Unter einer Grenz-Hyperebene ist hierbei eine beliebige Grenze zu verstehen, die den betrachteten Parameterraum in Bereiche aufteilt. Hierbei kann es sich um gerade bzw. flächige Hyperebenen handeln, wie beispielsweise eine Grenzlinie in einem zweidimensionalen Parameterraum oder eine Fläche in einem dreidimensionalen Parameterraum. Es kann sich aber auch um eine beliebig geformte Hyperebene bzw. Hyperfläche handeln, d. h. um eine abgewinkelte oder gekrümmte Hyperebene bzw. Hyperfläche, insbesondere auch eine in sich geschlossene Hyperebene/Hyperfläche, beispielsweise die Oberfläche einer Kugel oder eines Quaders in einem dreidimensionalen Parameterraum. Solche Grenz-Hyperebenen können beispielsweise eine Charakterisierung bzw. Klassifizierung eines Objekts (ein Organ, eine Struktur, ein Gewebe etc.) erleichtern, indem auf einfache Weise festgestellt werden kann, ob die Wertetupel, welche zu bestimmten Bildpunkten gehören, in einem bestimmten Teil des Parameterraums liegen, und somit eine Aussage darüber getroffen werden kann, ob das durch die Bildpunkte repräsentierte Objekt in eine bestimmte Klasse fällt bzw. in bestimmter Weise charakterisiert werden kann.

Wie oben bereits erwähnt, erfolgt nach einer weiteren bevorzugten Methode eine Analyse derart, dass sie eine Zuordnung der Wertetupel zu Wertetupel-Gruppen umfasst, um ihre Eigenschaften isoliert weiter zu untersuchen, d. h. es wird eine Clusterbildung durchgeführt. Diese Clusterbildung erfolgt bevorzugt vollautomatisch oder teilautomatisch, kann aber auch manuell erfolgen.

Besonders bevorzugt kann bei der Analyse auch eine Anordnung einer Wertetupel-Gruppe oder mehrerer Wertetupel-Gruppen bzw. Cluster im Parameterraum berücksichtigt werden. Die Analyse der Anordnung kann dabei die Lage und die Ausdehnung bzw. Form umfassen.

Dabei kann bevorzugt die Anordnung der Wertetupel-Gruppe auch unter Berücksichtigung einer kollektiven Position der Wertetupel im Parameterraum bestimmt werden bzw. definiert sein. Eine solche kollektive Position kann z. B. die Position eines Schwerpunkts, eines Mittelpunkts etc. der Wertetupel-Gruppe sein.

Des Weiteren kann die Analyse der Wertetupel oder auch der Wertetupel-Gruppen unter Berücksichtigung einer Position in Bezug zu zumindest einem Referenz-Wertetupel, insbesondere auch einer Referenz-Wertetupel-Gruppe, erfolgen. Die Referenz-Wertetupel bzw. Referenz-Wertetupel-Gruppen können dabei auf einer Datensammlung von Referenzmessungen basieren, beispielsweise Messungen an bestimmten Testgruppen, z. B. Patienten mit bestimmten Krankheitsbildern und gesunden Probanden.

Weiterhin kann die Analyse der Wertetupel eine Segmentierung innerhalb des Parameterraums umfassen. Bei dieser Segmentierung können beispielsweise Bereiche manuell durch Einschränkung auf bestimmte Parameter-Intervalle definiert werden, was auch als eine hochdimensionale Würfel-Segmentierung oder hochdimensionale Ellipsoide angesehen werden kann. Sofern der Parameterraum bereits grafisch einfach darstellbar ist, ist es möglich, dass dies auch manuell erfolgt, indem ein Befunder eine bestimmte Gruppe von Wertetupeln durch Ziehung von Grenzlinien separiert. Eine solche Segmentierung entspricht also im Prinzip der weiter oben beschriebenen Festlegung von Grenz-Hyperebenen innerhalb des Parameterraums. Soll eine Freihandsegmentierung erfolgen, kann diese aber auch in einer zwei- oder dreidimensionalen visuellen Darstellung des Parameterraums interaktiv eingezeichnet und automatisch auf den ursprünglichen Parameterraum extrudiert werden. Eine solche automatische Extrusion kann auch durch die automatische Selektion eines am dichtesten besetzten Parameterbereichs in der nicht sichtbaren (projizierten) Dimension ersetzt werden. Dies wäre analog zu einem "Correlated Cursor" auf einer räumlich-temporalen MIP: Bei diesem Verfahren wird durch Klick in ein auf 2D-projiziertes Bild automatisch ein Punkt aus dem vierdimensionalen Raum zugeordnet. Das zweidimensionale Bild zeigt hierbei die räumliche und temporale Projektion, d.h. es wird zunächst für jede Voxel-Position die höchste Signalintensität im zeitlichen Verlauf bestimmt und anschließend in dieser resultierenden (dreidimensionalen) temporalen MIP die höchste Signalintensität entlang der räumlichen Blickrichtung. Durch den Klick in dieses zweidimensionale Bild wird zuerst die räumliche Lage (x,y,z) in der dreidimensionalen temporalen MIP bestimmt (Position des Maximums entlang der Blickrichtung) und daraufhin die zeitliche Koordinate (Position des Maximums entlang der Zeitachse für den Punkt (x, y, z).

Eine weitere Möglichkeit ist eine halbautomatische oder automatische Segmentierung auf Basis von Schwellenwerten oder durch die oben beschriebene Clusterung, also z. B. eine Anwendung von Cluster-Detektionstechniken wie k-means-Clustering, Fuzzy-C-means-Clustering oder auch ein Regionenwachstumsverfahren. Ebenso ist ein Fitten von parametrischen Repräsentationen wie z. B. eine "Expectation Maximization" für Gaussians Mixture Models möglich.

Die Analyse kann auch morphologische Operationen und/oder Filterungen umfassen. Unter morphologischen Operationen sind hier beispielsweise Opening-Operationen, Closing-Operationen, Erosions-Operationen, Dilatationen und/oder Glättungen zu verstehen, wie sie bereits in der Bildverarbeitung eingesetzt werden.

Auch ist es möglich, bestimmte Bereiche durch Boolesche Kombination von Teilbereichen zu definieren, wie z. B., dass eine Region dadurch definiert ist, dass sie zu einer anderen Region gehört, jedoch nicht zu einer dritten Region.

Vorzugsweise kann auch eine zeitliche Analyse durchgeführt werden, welche eine Analyse der Veränderung von Wertetupeln im Parameterraum, vorzugsweise eine zeitliche Verschiebung einzelner Wertetupel oder der Anordnung bzw. kollektiven Position von Wertetupel-Gruppen, umfasst. Beispielsweise kann die Verschiebung des Mittelwerts eines Clusters über Untersuchungszeitpunkte hinweg ein klinischer Indikator für Therapieansprechen sein.

Besonders bevorzugt kann die Analyse ein maschinelles Lernverfahren umfassen bzw. es können in der Analyse maschinelle Lernverfahren genutzt werden. Insbesondere bieten sich Machine-Learning-Techniken zur Klassifizierung bzw. Charakterisierung an. Dies gilt besonders bei Verfahren unter Nutzung von Referenz-Wertetupeln, da im Rahmen der Klassifizierung bzw. Charakterisierung von Messwerten bzw. Untersuchungsobjekten mit jeder Analyse neue Daten in der Datenbank hinzugenommen werden können, die - gegebenenfalls nach einer Verifizierung durch andere Untersuchungen - wiederum als Referenz-Wertetupel bzw. Referenz-Wertetupel-Gruppen (bzw. Referenzcluster) genutzt werden können.

Insbesondere können die genannten hochdimensionalen, multiparametrischen Patientendaten bzw. medizinischen Messdaten auch mit Hilfe von Machine-Learning-Techniken analysiert werden, um Muster zu identifizieren, die z. B. einer Klassifikation in klinisch relevante Klassen (z. B. benigner, maligner Tumor bei erwartetem Tumor-Genotyp) oder einer Regression in kontinuierliche Parameterwerte (z. B. eine Wahrscheinlichkeit für Malignität; ein Surrogat für konventionelle Laborwerte) entsprechen können. Vorzugsweise kann auch durch multiparametrische Daten von mehreren Untersuchungsobjekten bzw. Patienten, bei denen die (klinische) Klassifizierung bekannt ist, ein Klassifikator für die Vorhersage der Zuordnung von neuen Patientendaten trainiert werden. D. h. die Daten werden, wie oben erläutert, als Referenzdaten für die nachfolgenden Analysen benutzt. Dabei kann bevorzugt eine Entscheidungsregel des Klassifikators auch extrahiert werden, um zukünftig dann als Entscheidungskriterium in der manuellen bildbasierten Befundung zu fungieren. Beispielsweise können Entscheidungsbäume für Klassifizierungen automatisch erlernt werden. Für die Machine-Learning-Techniken können auch künstliche neuronale Netze eingesetzt werden.

Bei der grafischen Darstellung des Parameterraums können höhere Dimensionen auch durch erweiterte Visualisierungstechniken einbezogen werden, wie z. B. Abbildung auf eine Farbskala oder Beschreibung durch Vektoren/Glyphen. Beispielsweise können mehrere Untersuchungszeitpunkte in einem 2D-Scatter-Plot in unterschiedlichen Farben dargestellt werden oder die Verschiebung einer Punktwolke (Cluster) kann durch einen Vektor im Scatter-Plot dargestellt werden.

Vorzugsweise umfasst die Visualisierung der Ergebnisdaten eine, besonders bevorzugt farbliche, Markierung von Bildpunkten und/oder Regionen in einer Ortsraumdarstellung, d. h. in einem anatomischen Bild des Untersuchungsobjekts. Die Markierung kann dabei in Abhängigkeit von einem Ergebnis der Analyse der Wertetupel in dem ersten und/oder einem weiteren Parameterraum erfolgen, insbesondere auch einer eventuellen zeitlichen Analyse. Anders gesagt, können die hochdimensionalen oder auf einen niedrigdimensionalen Raum projizierten Parameterwerte durch ein sogenanntes Back-Mapping oder erweitertes Back-Mapping zur Visualisierung mit den anatomischen Bilddaten fusioniert werden. Dabei können neben Histogramm-Einfärbungen auch semantische Klassen wie z. B. benigne oder maligne Regionen oder Wahrscheinlichkeitswerte für die Zugehörigkeit zu einer bestimmten Klasse durch die, insbesondere farbige, Markierung visualisiert werden. Insbesondere kann auch - wie oben bereits erwähnt - die zeitliche Analyse durch Farben oder Vektoren visualisiert werden, z. B. eine zeitliche Verschiebung als Vektor im Scatter-Plot.

Besonders bevorzugt können bei der Visualisierung der multiparametrischen Daten auch aus vorherigen Studien bekannte Modelle oder Ergebnisse als Referenzen in die Parameterräume mit eingezeichnet werden. Beispielsweise können manuelle oder (semi-) automatisch definierte Parameterbereiche wie hochdimensionale Rechtecke, Ellipsoide oder Hyperebenen oder andere Entscheidungsgrenzen den Daten grafisch überlagert werden.

Zusammengefasst werden also durch das erfindungsgemäße Verfahren folgende Vorteile erreicht:
- Reduktion der Daten-Dimensionalität,
- erweiterte Visualisierungstechniken und Back-Mapping,
- Segmentierungstechniken für bestimmte Bereiche in hochdimensionalen Parameterräumen,
- Regressions- und Klassifikationstechniken zur automatischen Analyse multiparametrischer Daten,
- Extraktion von neuen Entscheidungsregeln für konventionelle bildbasierte Befundung.

Somit kann das Potential der erfindungsgemäßen Bearbeitung von multiparametrischen Messdaten zu einer verbesserten Diagnose und Behandlung und im Weiteren dann auch zu Kostenersparnissen sowie zu optimierten Untersuchungs- und Behandlungspfaden ausgenutzt werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Es zeigen:
FIG 1 ein Ablaufschema für ein Verfahren gemäß einem Ausführungsbeispiel der Erfindung,
FIG 2 eine schematische Darstellung eines Beispiels einer Punktwolke in drei Parametern sowie hierzu ermittelte Hauptachsen,
FIG 3 eine schematische Darstellung der auf die Hauptachsen transformierten Punktwolke aus Figur 2 mit reduzierter Dimensionalität,
FIG 4 ein vereinfachtes Beispiel für eine Segmentierung von drei Regionen in dem reduzierten Parameterraum gemäß FIG 3,
FIG 5 ein vereinfachtes Beispiel für eine Markierung von Bildpunkten in einem anatomischen Bild bzw. einer Ortsraumdarstellung des Untersuchungsobjekts entsprechend ihrer Regionszugehörigkeit zu den Regionen in FIG 4 (Back-Mapping auf die morphologischen Ursprungsvoxel),
FIG 6 ein vereinfachtes Beispiel für eine Bestimmung von Untergruppen durch automatisches Clustern,
FIG 7 ein vereinfachtes Beispiel für eine mögliche bildbasierte Befundung anhand von Schwellwerten (Grenzlinien),
FIG 8 eine schematische Darstellung eines Beispiels für markierte interessierende Regionen (ROIs) in einem Magnetresonanz-Schnittbild durch eine Prostata,
FIG 9 ein Beispiel für unterschiedliche Zeitkurven bei einer dynamischen Kontrastmittel-Magnetresonanzmessung der Prostata in den in Figur 8 markierten interessierenden Regionen,
FIG 10 ein Beispiel für eine Clusterbildung und Segmentierung mit Hilfe von Grenzlinien in einem zweidimensionalen Parameterraum für Messwerte aus einer Messung gemäß FIG 9,
FIG 11 ein Beispiel für eine mögliche Klassifikation mithilfe von linearen Entscheidungsgrenzen,
FIG 12 eine schematische Darstellung eines medizinischen Datennetzwerks mit mehreren daran angeschlossenen Modalitäten und einer daran angeschlossenen Einrichtung zur Ermittlung von Ergebnisdaten auf Basis von medizinischen Messdaten aus verschiedenen Messungen gemäß einem Ausführungsbeispiel der Erfindung.

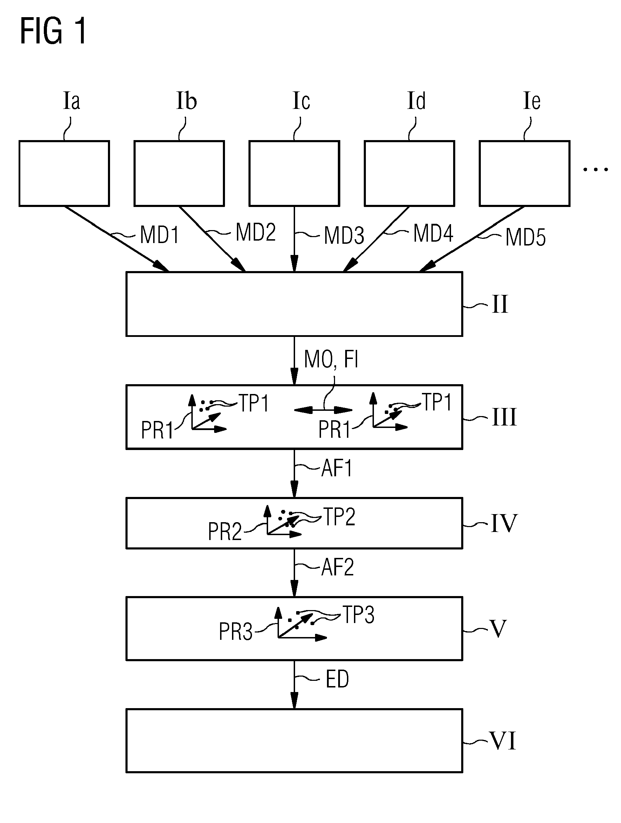

Bei dem in Figur 1 dargestellten beispielhaften Verfahrensablauf werden zunächst aus unterschiedlichen Messungen Ia, Ib, Ic, Id, Ie im Verfahrensschritt II Messdaten MD1, MD2, MD3, MD4, MD5 übernommen. Diese Messungen Ia, Ib, Ic, Id, Ie können beispielsweise an unterschiedlichen Geräten durchgeführt werden, z. B. im Verfahrensschritt Ia an einem MRT-Gerät, im Verfahrensschritt Ib an einem CT-Gerät, im Verfahrensschritt Ic an einem Ultraschallgerät, in einem Verfahrensschritt Id an einem PET-Gerät und in einem Verfahrensschritt Ie handelt es sich z. B. um die Übernahme bzw. Eingabe von Laborwerten wie beispielsweise PSA-Werten oder anderen Blutwerten. Grundsätzlich können auch weitere Geräte bzw. Messdaten wie EKG-Daten etc. hinzukommen. Ebenso können die unterschiedlichen Messungen aber auch an ein und demselben Gerät durchgeführt werden, wobei unterschiedliche Parameter ausgewertet werden, beispielsweise bei einer MRT-Messung unterschiedliche Kontraste etc., aber auch mit denselben oder sehr ähnlichen Parametern, jedoch zu unterschiedlichen Zeitpunkten und/oder Bedingungen wie mit oder ohne Kontrastmittel.

Bei diesen Messdaten MD1, MD2, MD3, MD4, MD5 handelt es sich in vielen Fällen um Bilddaten bzw. Parameterkarten. Dabei kann es sich um zweidimensionale Daten, um dreidimensionale Daten wie z. B. Volumendaten oder sogar um vierdimensionale Daten handeln, beispielsweise Volumendaten, die in bestimmten Zeitabständen aufgenommen wurden, so dass die vierte Dimension durch die Zeit dargestellt wird.

Im Verfahrensschritt II kann dann eine Voranalyse bzw. Zusammenstellung der Daten zu Wertetupeln TP1 für einen hochdimensionalen Parameterraum PR1 durchgeführt werden, d. h. die Messwerte können zu Vektoren zusammengestellt werden, wobei beispielsweise ein Vektor die Messdaten eines Bildpunkts (Pixel oder Voxel) enthält, ggf. auch zu unterschiedlichen Zeiten. Ebenso kann aber ein Wertetupel auch jeweils einen Wert zu unterschiedlichen Zeiten für eine Gruppe von Bildpunkten, ggf. auch für ein bestimmtes Organ oder eine bestimmte Region, umfassen.

Der hochdimensionale erste Parameterraum PR1, in dem die Wertetupel TP1, welche aus den einzelnen Messwerten der Messdaten MD1, MD2, MD3, MD4, MD5 gebildet ist, wird dann im Schritt III analysiert. Diese Analyse kann, wie dies schematisch dargestellt ist, auch Datenfilterungen FI und/oder morphologische Operationen MO umfassen, d. h. es findet eine Art Vorverarbeitung innerhalb des hochdimensionalen ersten Parameterraums PR1 statt.

Die Analyse im hochdimensionalen Parameterraum PR1 umfasst aber insbesondere eine Abbildung mit Hilfe einer ersten Abbildungsfunktion AF1 in einem niedriger dimensionierten Parameterraum PR2. Beispielsweise kann hier ein Parameterraum PR1 mit mehr als zwanzig Dimensionen in einen Parameterraum PR2 mit beispielsweise fünf oder sechs Dimensionen abgebildet werden. Dies hängt lediglich von der Wahl der Abbildungsfunktion AF1 ab. Beispiele hierfür werden später noch gegeben.

Im Verfahrensschritt IV findet dann eine Analyse der durch die erste Abbildungsfunktion AF1 erhaltenen Wertetupel TP2 im zweiten, niedriger dimensionierten Parameterraum PR2 statt. Hierbei können die gleichen Analysemethoden verwendet werden, wie sie im Schritt III in dem höher dimensionierten Parameterraum PR1 genutzt wurden, natürlich entsprechend angepasst an die geringeren Dimensionen. In der Regel handelt es sich aber um andere Analysemethoden.

Beispielsweise könnte in der ersten Stufe im Schritt III eine Clusterung in Wertetupel-Gruppen hinsichtlich bestimmter Dimensionen des ersten Parameterraums PR1 erfolgen. Die Abbildungsfunktion AF1 kann dann so gewählt sein, dass eine Anzahl von Parameterwerten, die genau diese Dimensionen, hinsichtlich derer die Clusterbildung erfolgt ist, einfach durch einen Parameterwert ersetzt wird, welcher die Zuordnung zu einem der Cluster repräsentiert. Auf diese Weise kann eine erhebliche Dimensionsreduzierung erreicht werden. In der weiteren Stufe kann bei der Analyse im niedriger dimensionierten Parameterraum PR2 dann wieder eine Unterklassifizierung oder dergleichen erfolgen. Hierzu kann beispielsweise geprüft werden, wo sich die neuen reduzierten Wertetupel TP2 hinsichtlich einer Grenz-Hyperebene befinden. Prinzipiell erfolgt auch bei dieser weiteren Analyse im zweiten Parameterraum PR2 eine Abbildung mittels einer (weiteren) Abbildungsfunktion AF2 in einen dritten, noch niedriger dimensionierten Parameterraum PR3, in dem die Wertetupel dann durch entsprechende Wertetupel TP3 repräsentiert werden. Im weiteren Parameterraum PR3 kann dann wieder eine beliebige Analyse und Weiterreduzierung, vorzugsweise auch mit einer Abbildungsfunktion, auf einen weiteren, noch niedriger dimensionierten Parameterraum erfolgen.

In Figur 1 ist diese Variante nicht dargestellt, sondern hier ist bereits im Schritt V eine Stufe erreicht, bei der bei der Analyse im letzten Parameterraum PR3 sofort die Ergebnisdaten ED ermittelt werden, die dann im Schritt VI ausgegeben bzw. visualisiert werden. Grundsätzlich wären aber auch sogar mehr als drei Stufen möglich.

Eine Ausgabe bzw. Visualisierung kann beispielsweise ein Back-Mapping in einen Ortsraum des Untersuchungsobjekts, also beispielsweise Überlagerung in einem anatomischen Bild etc., umfassen. Zusätzlich können diese Ergebnisdaten aber auch beispielsweise nur die Ausgabe einer einzelnen Klassifizierung bzw. Charakterisierung des Untersuchungsobjekts, also z. B. des Patienten oder eines Organs oder eines bestimmten Gewebebereichs des Patienten, umfassen. Es wird an dieser Stelle darauf hingewiesen, dass die Ergebnisdaten ED bei einer Klassifizierung in der Regel nur Befundungsvorschläge sind, die in der Regel vom Befunder noch bestätigt werden können bzw. bestätigt werden müssen. Ebenso ist es auch möglich, dass die Klassifizierung bzw. Charakterisierung zusätzlich mit Angaben über Wahrscheinlichkeiten erfolgt, mit denen das Untersuchungsobjekt bzw. Organ oder Gewebeteile dieser Klassifizierung bzw. Charakterisierung entsprechen.

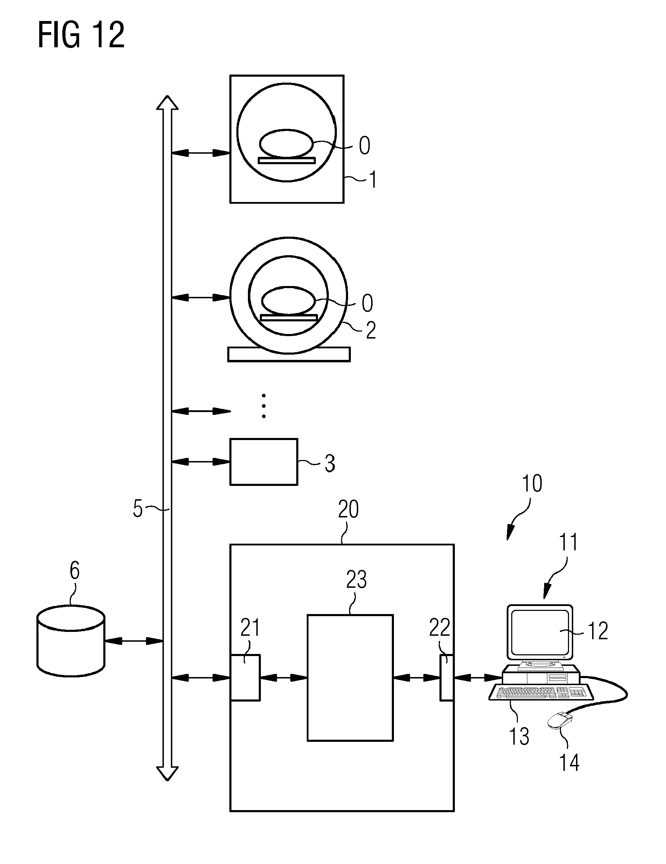

In Figur 12 ist schematisch eine Einrichtung 10 dargestellt, mit der ein solches Verfahren realisierbar ist. Gezeigt ist hier ein medizinisches Netzwerk, beispielsweise ein radiologisches Informationssystem (RIS), in dem verschiedene Geräte über einen Datenbus 5 miteinander gekoppelt sind. Im vorliegenden Fall handelt es sich zum einen um Modalitäten 1, 2, 3, an denen Messungen am Untersuchungsobjekt O erfolgen und von denen Messdaten übernommen werden können, einen Massenspeicher 6, um Messdaten, insbesondere Rohdaten, Bilddaten etc. zu hinterlegen, sowie die erfindungsgemäße Einrichtung 10 zur Ermittlung der Ergebnisdaten auf Basis der medizinischen Messdaten. Zusätzlich können an dieses Netz bzw. den Datenbus 5 noch weitere Geräte angeschlossen sein, insbesondere auch eine Schnittstelle zu einem externen Netz, beispielsweise dem Internet etc.

In dem dargestellten Beispiel handelt es sich bei einem der Modalitäten 1 um ein kombiniertes MRT-PET-Gerät 1. Bei einer anderen Modalität 2 handelt es sich um einen Computertomographen 1. Lediglich symbolisch ist eine weitere Modalität 3 dargestellt, beispielsweise ein Ultraschallgerät, ein Röntgengerät etc. Ebenso könnte hier eine weitere Schnittstelle zur Übernahme von Laborwerten etc. vorgesehen sein, um diese zusätzlich innerhalb des Verfahrens zu übernehmen und zu verwenden.

Die erfindungsgemäße Einrichtung 10 umfasst hier zum einen eine Rechnereinheit 20, welche über eine Datenschnittstelle 21 an den Datenbus 5 und über eine weitere Datenschnittstelle 22 an ein Terminal 11 zur Bedienung der Einrichtung 10 angeschlossen ist. Außerdem umfasst die Recheneinheit 20 ein entsprechendes Softwaremodul 23 mit den erforderlichen Softwarekomponenten, um die datenverarbeitungstechnischen Maßnahmen und Berechnungen auszuführen, um das erfindungsgemäße Verfahren durchzuführen. Weiterhin umfasst die Rechnereinheit 20 üblicherweise eine (hier nicht dargestellte) geeignete Speichereinrichtung (Festplatte(n), RAM etc.), auf der die notwendigen Softwarekomponenten gespeichert sind.

Das Terminal 11 umfasst eine Ausgabeeinheit 12, hier symbolisiert durch einen einfachen Bildschirm, sowie Eingabeeinheiten, wie beispielsweise eine Tastatur 13 und/oder ein Zeigegerät 14, hier symbolisiert durch eine Maus. Selbstverständlich kann der Bildschirm 12 auch als Touchscreen oder dergleichen ausgestaltet sein. Die gesamte Einrichtung 10, also die Rechnereinheit 20 zusammen mit dem Terminal 11 bzw. der Benutzerschnittstelle 11, kann beispielsweise in Form einer üblichen Workstation zur Befundung in einem medizinisches Datennetzwerk realisiert sein.

Über das Terminal kann der Befunder bestimmte Daten auswählen, sich diese anzeigen lassen und/oder mit Hilfe einer grafischen Benutzerschnittstelle, nämlich Bildschirm 12 sowie Tastatur 13 und Maus 14 oder anderen entsprechenden Hilfsmitteln gegebenenfalls Markierungen, Grenzlinien setzen und Segmentierungen vornehmen etc., wie nachfolgend noch erläutert wird. Ebenso können Befehle zur Speicherung von Daten, zur Konfiguration der Recheneinheit 20 hinsichtlich der gewünschten Analysemethoden bzw. der Dimensionen der Parameterräume etc. erfolgen. Mit anderen Worten, alle Benutzeraktionen für im Rahmen des Verfahrens durch einen Benutzer zu unterstützende bzw. zu initiierende Operationen können beispielsweise mit Hilfe dieses Terminals durchgeführt werden.

Im Folgenden soll anhand der weiteren Figuren mit Hilfe von einfachen Beispielen die prinzipielle Vorgehensweise hinsichtlich bestimmter Analysetechniken, Abbildungsfunktionen bzw. Visualisierungsmethoden erläutert werden. Sofern Figuren hierzu herangezogen wurden, sind die gegebenen Beispiele wegen der Darstellbarkeit auf drei Dimensionen begrenzt, in der Regel sind diese Operationen aber auch in den hochdimensionalen Parameterräumen durchführbar.

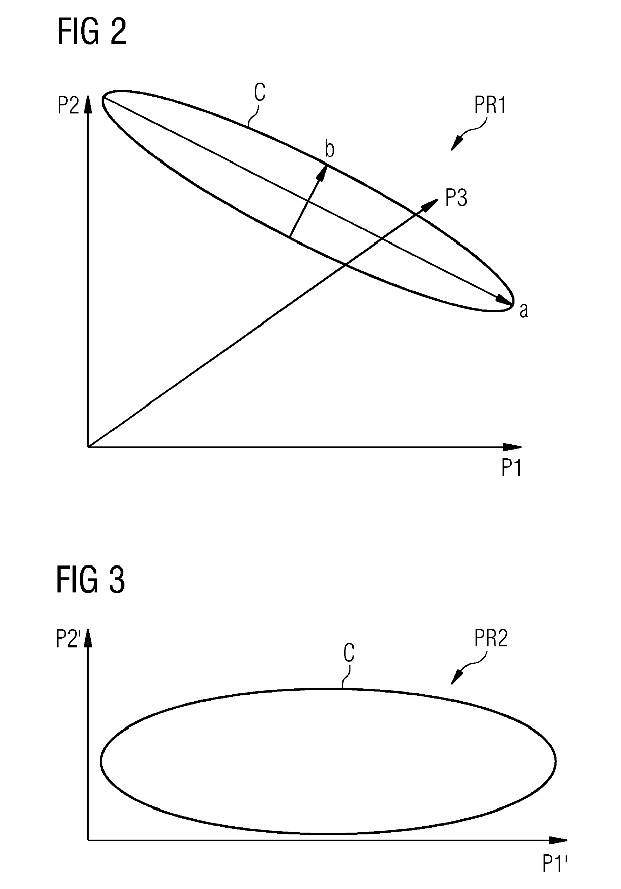

Anhand der Figuren 2 und 3 ist beispielsweise ein Verfahren dargestellt, wie sich eine Reduktion der Datendimensionalität mit Hilfe einer Hauptachsentransformation durchführen lässt. Figur 2 zeigt schematisch eine angepasste Einhüllende einer Punktwolke (selbst nicht dargestellt), also beispielsweise einen Cluster von Wertetupeln, in einem dreidimensionalen Raum mit den Koordinatenachsen P1, P2, P3. Diese Koordinatenachsen P1, P2, P3 sind die Parameter, welche durch den entsprechenden Parameterwert im Wertetupel repräsentiert werden.

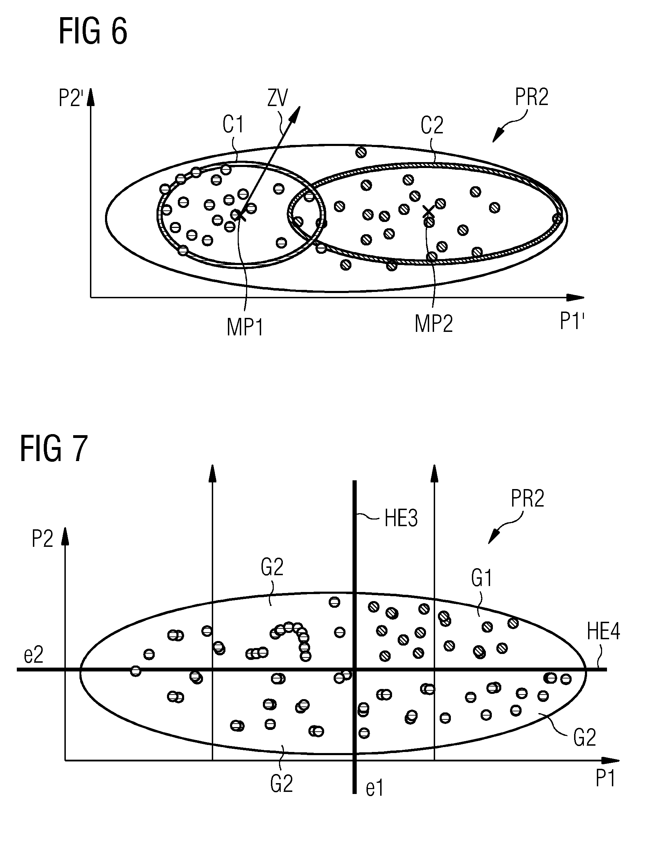

Anhand von Figur 6 wird später noch dargestellt, wie eine solche Clusterung der Wertetupel erfolgen kann.

In der hier in Figur 2 gezeigten dreidimensionalen Betrachtung lässt sich ein solcher Cluster bzw. die Punktwolke von Interesse zwar noch visuell erfassen. Für eine Quantifizierung müssen jedoch auch hier schon alle drei Ausgangsparameter bzw. Koordinaten P1, P2, P3 betrachtet werden. Oftmals sind die Daten auf niedrigdimensionale Unterräume beschränkt, wie im folgenden Beispiel eine planare oder nahezu planare Ellipse. Durch eine übliche Hauptachsentransformation können in diesem dreidimensionalen Raum die Hauptachsen a, b und geeignete Koordinaten ermittelt werden. Die Punktwolke bzw. der Cluster C kann dann dementsprechend in einem auf zwei Dimensionen reduzierten Parameterraum PR2 mit den Koordinatenachsen P1`, P2' dargestellt werden, wobei eine Koordinatenachse P1` in Richtung der einen Hauptachse a verläuft und die andere Koordinatenachse P2` in Richtung der anderen Hauptachse b der Ellipse. Bei einer Reduzierung von drei auf zwei Dimensionen ist dies noch, wie in den Figuren 2 und 3, einfach darstellbar. Es ist aber klar, dass dieses Grundprinzip auf erheblich höher dimensionierte Räume übertragen werden kann. Durch die Reduktion der Dimensionalität kann dann die Punktwolke durch benutzerfreundliche Methoden, wie im vorliegenden Fall z. B. durch zweidimensionale Definition von interessierenden Regionen, durch Festlegung von Schwellenwerten etc., wesentlich leichter analysiert werden, als dies im ursprünglich höher dimensionalen Parameterraum möglich gewesen wäre.

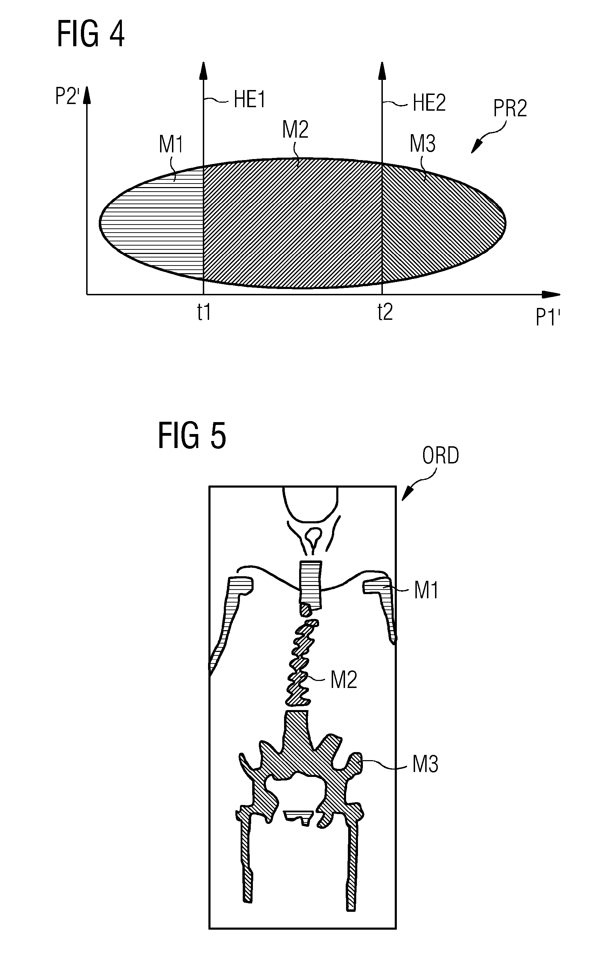

Dies wird im Folgenden anhand von Figur 4 an einem einfachen Beispiel verdeutlicht. Hier wird die Punktwolke (Cluster) aus Figur 4 mit Hilfe von zwei Schwellenwerten t1 und t2 in drei Bereiche aufgeteilt. Diese Schwellenwerte t1, t2 bilden hier quasi Hyperebenen HE1, HE2 des zweidimensionalen Parameterraums PR2. Die Wertetupel können somit unterschiedlichen Regionen in dem Parameterraum zugeordnet werden, hier alle Wertetupel unterhalb der Schwelle t1 des Parameters P1`, alle Wertetupel zwischen den Schwellen t1 und t2 bezüglich des Parameters P1` und alle Werte oberhalb des Schwellenwerts t2 hinsichtlich des Parameters P1`. Das Setzen der Schwellenwerte t1, t2 bzw. der Hyperebenen HE1, HE2 erfolgt sinnvollerweise natürlich gemäß einer bestimmten medizinischen Relevanz bzw. Aussage, beispielsweise "vermutlich gesundes Gewebe", "vermutlich Gewebe eines malignen Tumors" oder "vermutlich Gewebe eines benignen Tumors". Auch dieses Prinzip ist auf einen beliebig höher dimensionalen Raum übertragbar.

Anschließend ist ein Back-Mapping der Regionszugehörigkeit der Wertetupel, z. B. durch eine Farbkodierung, auf die morphologischen Ursprungsvoxel möglich, wie dies in Figur 5 dargestellt wird. Mit anderen Worten, es wird hier jedem Wertetupel, welcher ja für einen bestimmten Bildpunkt des ursprünglichen Bildes steht bzw. diesen repräsentiert, aufgrund der Regionszugehörigkeit im Raum PR2 gemäß Figur 4 ein Farbwert zugeordnet und mit diesem Farbwert erfolgt dann eine Markierung des entsprechenden Bildpunkts, d. h. Pixels oder Voxels, in der Ortsraumdarstellung ORD. Durch diese farbigen Markierungen M1, M2, M3 kann so unmittelbar in der Ortsraumdarstellung ORD vom Befunder erkannt werden, welcher Region bzw. welchen Klassen die entsprechenden Bildpunkte innerhalb des Parameterraums PR2 in Figur 4 zuzuordnen sind. Die Markierung kann insbesondere auch durch eine Überlagerung einer Maske mit entsprechenden Markierungen der Bildpunkte mit einem anatomischen Bild erfolgen.

Figur 6 zeigt eine andere Möglichkeit, wie anstelle einer Schwellenwertsetzung wie in Figur 4 eine Analyse einer Punktwolke im dimensionsreduzierten Raum erfolgen könnte. Hier ist als Beispiel dargestellt, wie mit Hilfe von automatischen Clustering-Algorithmen wiederum Untergruppen bzw. Untercluster C1, C2 innerhalb der Punktwolke bzw. des großen Clusters definiert werden. Für diese Cluster C1, C2 bzw. Untercluster C1, C2 können wie für jeden Cluster auch kollektive markante Punkte festgestellt werden, hier z. B. Mittelpunkte MP1, MP2 oder Schwerpunkte der Cluster C1, C2 oder andere "Cluster-Zentren".

Sofern eine zeitliche Analyse erfolgt, d. h. in einer weiteren Dimension die Anordnung der einzelnen Wertetupel über der Zeit innerhalb der jeweils betrachteten Parameterräume möglich ist (indem beispielsweise einfach eine Zeitachse hinzugenommen wurde), kann nach Auswertung des zeitlichen Verhaltens auch eine Verschiebung eines solchen Clusters C1, C2 im Raum markiert bzw. angezeigt werden. Eine solche zeitliche Verschiebung ZV kann auch durch einen Vektor dargestellt werden, in welche Richtung sich beispielsweise der kollektive Punkt, hier der Mittelpunkt MP1 eines Clusters C1, mit der Zeit bewegt.

Eine Anzeige des zeitlichen Verhaltens ist oft sinnvoll, da auch die zeitliche Varianz eine medizinisch relevante Aussage enthalten kann. Beispielsweise kann dies insbesondere dann der Fall sein, wenn es sich um unterschiedliche Messungen zu zeitlich weiter beabstandeten Zeitpunkten handelt, z. B. im Rahmen von Verlaufs- oder Folgemessungen zur Überprüfung eines Therapieerfolgs. Auf diese Weise kann mit Hilfe eines Vektors für die zeitliche Verschiebung ZV eines Clusters C1 recht gut visualisiert werden, ob eine Therapie anschlägt oder nicht.

Insbesondere ist es auch möglich, die detektierten Cluster-Zentren in einer Patientenpopulation dafür zu nutzen, für einen neuen Patientenfall eine Wahrscheinlichkeit für eine bestimmte Klassenzugehörigkeit zu bestimmen, d. h. es können beispielsweise dann die hier durchgeführten Analysen bzw. die dabei bestimmten kollektiven Werte oder Zentren (bzw. Mittelpunkte, Schwerpunkte etc.) von Clustern als Referenzwerte verwendet werden, um anhand dieser dann bei Analysen der Messwerte von anderen Patienten eine Klassifizierung bzw. Charakterisierung von Gewebe, Organen etc. durchführen zu können.

Des Weiteren ist es auch möglich, neue Entscheidungsregeln für konventionelle bildbasierte Befundungen mit Hilfe des erfindungsgemäßen Verfahrens zu extrahieren. Wenn sich z. B. im Rahmen des erfindungsgemäßen Verfahrens herausstellt, dass sich eine gesuchte Untergruppe auch im ursprünglichen Parameterraum durch einfache Schwellenwertbildung hinreichend genau abgrenzen lässt, kann dieses Ergebnis dann in eine bildbasierte Befundungsregel überführt werden.

Anhand von Figur 7 ist hier ein besonders einfaches Beispiel dargestellt. Hier ist im Rahmen von weiteren Analysen festgestellt worden, dass, wenn eine Voxelintensität bezüglich des Parameters P1 unterhalb eines Grenzwert e1, d. h. im Bild links von einer Hyperebene HE3, liegt und gleichzeitig die Voxelintensität hinsichtlich des Parameters P2 größer als ein Grenzwert e2 ist, d. h. im Bild oberhalb einer Hyperebene HE4 liegt, dieser Voxel in eine Gruppe G1 gehört, beispielsweise zu gesundem Gewebe. Andernfalls gehört er in die Gruppe G2, welche ein Gewebe mit einer Läsion indiziert.

Nachfolgend werden weitere konkretere Ausführungsbeispiele in vereinfachter Form für das erfindungsgemäße Verfahren erläutert.

### Beispiel 1: Differenzierung zwischen gesundem und krankem Gewebe mittels einer dynamischen Kontrastmittel-Perfusions-MRT

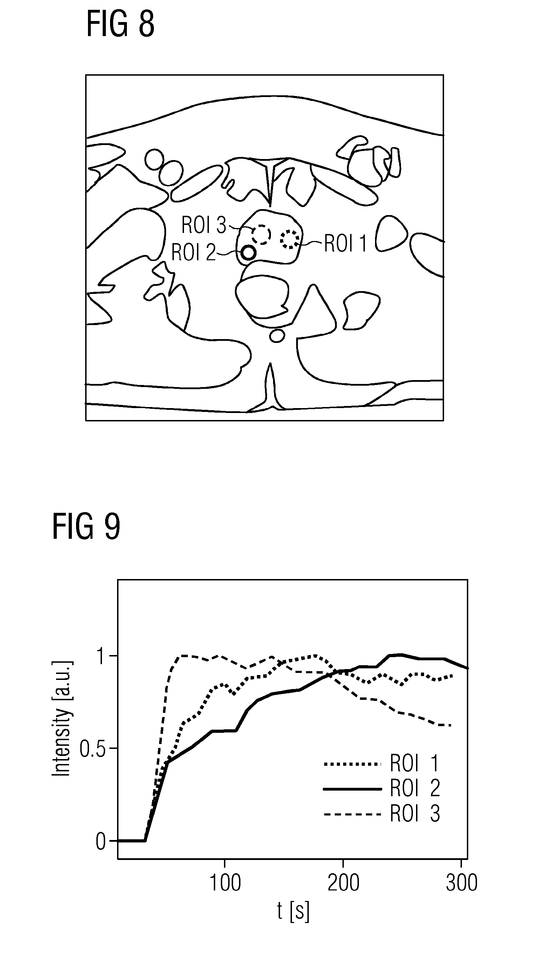

Bei diesem Beispiel, welches anhand der Figuren 8 bis 10 erläutert wird, handelt es bei den Messungen um dynamische Kontrastmittel-Perfusions-MRT-Messungen. Hierbei werden während einer Kontrastmittelgabe mehrere, typischerweise vier bis fünfzig, Volumina akquiriert. Das Anreicherungsverhalten des Kontrastmittels unterscheidet sich zwischen unterschiedlichen Gewebearten bzw. unterschiedlichen Organen und erlaubt somit auch die Differenzierung zwischen gesundem und krankem Gewebe (wie Tumoren). In Figur 8 sind hierzu unterschiedliche interessierende Regionen ROI1, ROI2, ROI3 in der Prostata schematisch dargestellt. Die Figur 9 zeigt hierzu die unterschiedlichen Zeitkurven bei einer solchen dynamischen Kontrastmittel-MRT in der Prostata. Hierzu sind in Figur 9 jeweils die Kurven der Kontrastmittelanreicherung (Intensität in willkürlichen Einheiten [a. u.]) über der Zeit t (in s) dargestellt, wobei jeweils ein Mittelwert für die unterschiedlichen Regionen ROI1, ROI2, ROI3 aus Figur 9 verwendet wird. In der Literatur oder in bestimmten auf dem Markt erhältlichen Produktlösungen existieren unterschiedliche Ansätze zur Analyse solcher Kontrastmittel-Dynamiken. Beispielsweise kann die Steigerung der Kontrastmittelanflutung ("Wash-in") oder - auswaschung ("Wash-out") in der Zeitkurve jedes Voxels berechnet werden.

Erfindungsgemäß kann der Verlauf der Bildintensität jedes einzelnen Bildpunkts, d. h. Voxels oder Pixels, über der Zeit als Parametervektor interpretiert werden. Damit ergibt sich bei p aufgenommenen zeitlichen Phasen für jeden Bildpunkt ein p-dimensionaler Vektor in einem p-dimensionalen Parameterraum. Im Rahmen der vorliegenden Erfindung kann nun ein solcher p-dimensionaler Parameterraum derart analysiert und mit Hilfe von Abbildungsfunktionen bearbeitet werden, dass eine effiziente Darstellung oder Nutzung möglich ist. Es wird in diesem Zusammenhang explizit darauf hingewiesen, dass die nachfolgend dargestellte Analyse von dynamischen Kontrastmittel-Perfusions-MRT-Daten auch für diffusionsgewichtete MRT-Daten oder allgemeine multiparametrische Bildgebungsdaten in analoger Weise durchgeführt werden kann.

Beispielsweise können im Rahmen von Trainingsmessungen oder dergleichen musterhafte Signalverläufe von Patienten mit z. B. histopathologisch bekannten Gewebeeigenschaften für verschiedene Klassen erstellt bzw. zur Verfügung gestellt werden, wie z. B. k = 5 Klassen "Tumor", "Nekrose", "gesundes Gewebe von Organ 1", "gesundes Gewebe von Organ 2", "Gefäß",. Mit Hilfe dieser Referenzdaten kann dann in dem p-dimensionalen Parameterraum für jede Signalkurve mit unbekannter Gewebeeigenschaft die Zugehörigkeitswahrscheinlichkeit zu jeder der fünf genannten Klassen geschätzt werden. Durch eine entsprechende Zuordnungsfunktion kann folglich die Dimension des Parameterraums von p auf k, hier also fünf Klassen, reduziert werden. Diese Dimensionsreduktion erfolgt dabei daten-unabhängig, da die Referenzkurven aus einer Bibliothek stammen, die vorab erstellt wurde.

Alternativ können aber in analoger Weise auch datenabhängige Dimensionsreduzierungen durchgeführt werden, indem ähnliche Zeitkurven einer Studie automatisch gruppiert und Clustern zugeordnet werden, ohne dass dabei zwingend externe Referenzkurven benutzt werden. Algorithmisch lässt sich so etwas beispielsweise durch k-nearest-neighbour-Klassifikationen realisieren.

Eine alternative datenabhängige Technik zur Dimensionsreduzierung ist auch hier wieder eine Hauptachsentransformation. Dabei könnte der Datenvektor für jeden räumlichen Punkt auf eine neue Basis abgebildet werden, in der die stärkste Variation in den ersten Dimensionen zusammengefasst wird. Auch dadurch ergibt sich die Möglichkeit, durch Verwerfen der höheren Dimensionen die Dimensionalität sinnvoll zu reduzieren. Jeder Bildpunkt kann, ausgehend von kontinuierlichen Klassenzugehörigkeitswahrscheinlichkeiten, auch einer bestimmten Klasse zugeordnet werden.

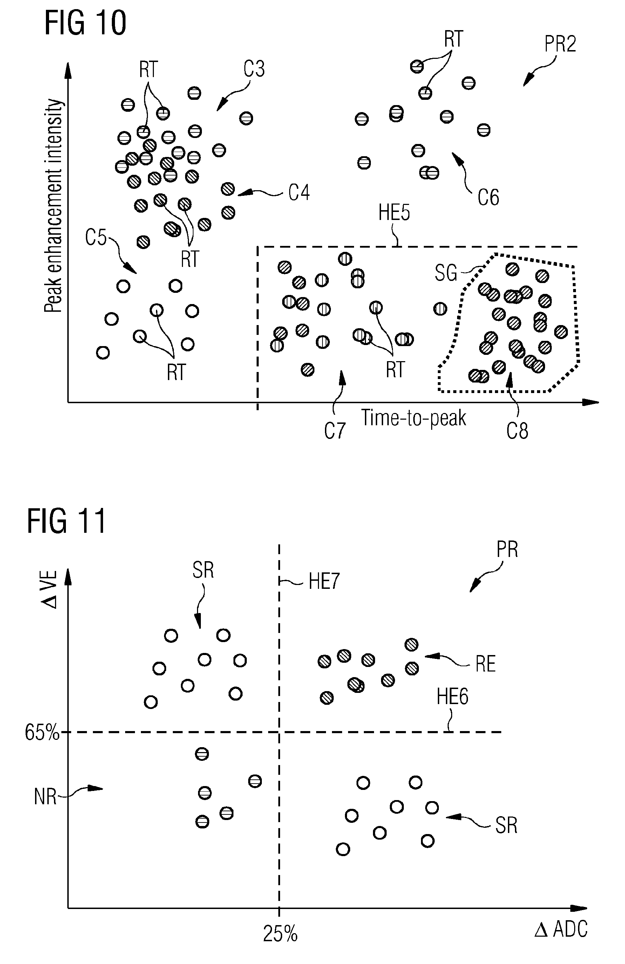

Weiterhin kann jeder Bildpunkt bezüglich einer anderen, niedrig-dimensionalen, anschaulicheren Basis visualisiert werden. In dem in Figur 10 dargestellten Diagramm wird für jeden Intensitätsvektor das Maximum der Kontrastmittelintensität (Peak Enhancement Intensity) sowie die zeitliche Position dieses Maximums (Time to Peak) als Basis für einen darstellbaren Vektorraum verwendet (in Figur 10 handelt es sich nur um eine schematische Darstellung mit willkürlichen Achsenskalierungen; auch sind die eingezeichneten Cluster hier willkürlich und nur aus Gründen der Anschaulichkeit so gewählt). Die Klassenzugehörigkeit zu einer bestimmten Klasse kann durch eine Farbe (oder wie in Figur 10 durch unterschiedliche Symbole) signalisiert werden. In diesem Diagramm bilden sich also insgesamt acht verschiedene Cluster aus, ein Cluster C3 für Bildpunkte, welche die Aorta darstellen, ein Cluster C4, welcher Tumorgewebe umfasst, ein Cluster C5 für gesundes Prostatagewebe, ein Cluster C6 für gesundes Blasengewebe und zwei Cluster C7, C8, welche beide nekrotisches Gewebe darstellen, da sie sich in einer Region in diesem Parameterraum PR befinden, welche durch eine abgeknickte Hyperebene HE5 bzw. eine Grenzlinie vom übrigen Bereich des Parameterraums PR2 abgegrenzt ist. Für die Clusterung können Referenz-Wertetupel RT aus vorherigen Studien genutzt werden (beispielhaft sind hier wenige Referenz-Wertetupel RT für verschiedene Cluster symbolisiert). Auch die Hyperebene HE5 kann beispielsweise aus vorherigen Studien bekannt sein. Es kann sich hierbei um einen oder mehrere sogenannte Entscheidungsgrenzen (Decision Boundaries) handeln, die hier u. a. beispielsweise durch die Erkenntnis begründet sein können, dass nekrotisches Gewebe typischerweise eine sehr langsame Kontrastmittelanflutung und ein sehr geringes Peak Enhancement aufweist. Solche Entscheidungsgrenzen können als Unterstützung mit den tatsächlichen Datenpunkten visualisiert werden.

Umgekehrt lassen sich, wie in Figur 10 auch dargestellt, visuell identifizierbare Entscheidungsgrenzen auf einfache konventionelle Entscheidungsregeln zurückübertragen. Im vorliegenden Fall beispielsweise, wenn der Parameter "Time to Peak" größer als die durch die Hyperebene HE5 definierte Schwelle und der Parameter "Peak Enhancement Intensity" kleiner als die durch die Hyperebene HE5 definierte Schwelle ist, das Gewebe höchstwahrscheinlich "nekrotisch" ist.

Außerdem können bekannte Cluster-Zentren oder entsprechende Wahrscheinlichkeitsdichteverteilungen angezeigt werden. In dem Beispiel in Figur 10 ist erkennbar, dass das nekrotische Gewebe sich in zwei Unter-Cluster C7, C8 gruppiert, die möglicherweise durch unterschiedliche Gewebeprozesse verursacht werden. Um einen möglichen räumlichen Zusammenhang zu überprüfen, kann der Befunder einen Teil der nekrotisch markierten Bildpunkte bzw. Wertetupel im Parameterraum selektieren. Dies ist z. B. durch eine Freihand-Segmentierung SG mit Hilfe der Benutzerschnittstelle bzw. des Terminals möglich. Eine solche Freihand-Segmentierung SG ist in Figur 10 durch die gepunktete Linie symbolisiert.

Die dadurch selektierten Wertetupel bzw. Bildpunkte können anschließend wiederum in einem Back-Mapping einem geeigneten anatomischen Bild überlagert werden. Es kann grundsätzlich aber auch gleichzeitig ein Back-Mapping aller Wertetupel bzw. Bildpunkte erfolgen, wobei jeder Punkt entsprechend seiner Klassenzugehörigkeit beispielsweise farbkodiert oder durch ein Symbol kodiert wird.

Auch bei den zuvor genannten konkreten Verfahren ist wieder eine Datenanalyse mit einem maschinellen Lernverfahren möglich. Für die akkurate klinische Diagnose und Therapie sind oftmals konkrete pathologische Befunde oder Biomarker (Blutserum-Werte etc.) ausschlaggebend. Durch Datenanalyse mit Hilfe eines Machine-Learning-Verfahrens lassen sich Computersysteme trainieren, eine automatische Abbildung von multiparametrischen medizinischen Bilddaten auf solche, in diesem Fall synthetische, Surrogat-Biomarker durchzuführen. Beispielsweise kann ein solches System durch automatische Analyse von Prostatakrebspatienten-Datensätzen mit gegebenen PSA-Werten und histopathologischen Befunden (Gleason-Score der Biopsie) sowie entsprechender multiparametrischer MRT-Bildgebung eine Korrelation zwischen den Bildparametern und dem Gleason-Score oder dem PSA-Wert erlernen. Dieses Korrelationsmodell kann dann nachfolgend angewendet werden, um für neue Patienten auf Basis der Bilddaten einen Gleason-Score-Wert vorherzusagen, ohne dass eine Biopsie durchgeführt werden müsste.

### Beispiel 2: Beurteilung des Therapieerfolgs bei intraarterieller Therapie durch MRT-Messungen

Insbesondere bei intraarteriellen Therapien bei Lebertumoren und Ähnlichem ist es ein Ziel, frühzeitig durch die Erkennung von Veränderungen in der MRT-Verlaufskontrolle festzustellen, ob die Therapie wirkt oder durch alternative Therapien ersetzt werden soll. Das Therapieansprechen kann beispielsweise klassifiziert werden in "Responder" (wirksam), "Semi-Responder" (teilweise wirksam) und "Non-Responder" (kein Ansprechen). Hierzu werden vor und nach der Therapie diverse MR-Kontraste inklusive kontrastmittelunterstützer Leber-Dynamik akquiriert, typischerweise jeweils ca. zwölf Volumina.

In der Literatur und in der Praxis existieren unterschiedliche Ansätze zur Analyse der Veränderungen. Hierzu kann beispielsweise für die Zielregion, d. h. den Tumor, der durchschnittliche ADC-Wert oder das durchschnittliche venöse Enhancement (VE) zu jedem Zeitpunkt ermittelt werden. Möglich wäre dabei eine Verwendung von einfachen Schwellwerten, um auf Basis der Veränderungen des durchschnittlichen ADC-Werts und des durchschnittlichen VE-Werts das Therapieansprechen zu bewerten.

Im Rahmen des erfindungsgemäßen Verfahrens wäre es zudem möglich, den Verlauf der Bildintensität sowie zusätzliche Attribute (räumliche Koordinaten, Akquisitionsparameter, Lebervolumen, Blutwerte wie AFP, Bilirubin etc.) für jeden einzelnen Bildpunkt als Parametervektor zu interpretieren. Beispielsweise ergäbe sich bei zwölf MR-Kontrasten und zwei Untersuchungszeitpunkten für jeden Bildpunkt in der Zielregion alleine daraus schon ein 24-dimensionaler Parametervektor. Anders als im Stand der Technik, bei dem ja nicht jeder Bildpunkt betrachtet wird, sondern nur der Mittelwert aller Bildpunkte einer Zielregion, wodurch räumliche Informationen, wie z. B. Heterogenität innerhalb des Tumors, verloren gehen, ist so eine vollständige Analyse möglich.

Um eine Dimensionsreduzierung zu erreichen, sind wieder die bereits oben genannten Verfahren verwendbar, beispielsweise kann hier auch eine Differenzberechnung als Dimensionsreduzierung genutzt werden, indem z. B. der ADC-Wert zu einem ersten Zeitpunkt abzüglich des ADC-Werts zu einem zweiten Zeitpunkt ermittelt wird und so zwei Werte innerhalb des Wertetupels durch einen Differenzwert ersetzt werden, um den Wertetupel um eine Dimension zu reduzieren. Eine weitere Möglichkeit wäre eine Dimensionsreduzierung auf eine neue Basis mit weniger Dimensionen mit Hilfe einer Hauptkomponentenanalyse, beispielsweise eine Reduktion von drei Parametern wie ADC, VE, Fat Fraction auf zwei Parameter.

Auch hier ist es wieder möglich, jeden einzelnen Bildpunkt oder das gesamte Tumorvolumen einer bestimmten wahrscheinlichsten Klasse unter den oben aufgeführten Klassen zuzuordnen, indem z. B. die Parameterwerte bzw. die Lage der einzelnen Wertetupel im hochdimensionalen Parameterraum mit entsprechenden Wertetupeln von Patienten mit bekanntem Therapieverlauf verglichen werden. D. h. hier wird auf Referenz-Wertetupel zurückgegriffen und so eine direkte Klassifikation der einzelnen Bildpunkte oder auch einer Region durchgeführt. Ebenso können aus vorherigen Studien bestimmte Entscheidungsgrenzen bekannt sein, d. h. Hyperebenen, die als Grenzflächen oder -linien dienen können, wie beispielsweise, dass sich der ADC-Wert oder VE-Wert mindestens um einen gewissen Prozentsatz verändert hat. Ein solches Beispiel ist in Figur 11 sehr einfach schematisch dargestellt. Aufgetragen ist hier ein Parameterraum, dessen eine Achse die Differenz der VE-Werte aus der ersten und zweiten Messung umfasst (ΔVE in %) und auf der anderen Achse die Differenz der ADC-Punkte aus den beiden Messungen (ΔADC in %). Auf Basis bekannter Studien können hier zwei Hyperebenen HE6, HE7 - in diesem Bild in Form von einfachen Grenzlinien - bestimmt werden, die den gesamten Raum in vier Quadranten aufteilen. Je nachdem, in welchem Bereich die Wertetupel angeordnet sind, sind sie einer der Klassen zuzuordnen, nämlich entweder der Klasse "Non-Responder" NR oder der Klasse "Semi-Responder" SR oder der Klasse "Responder" RE. Es ist klar, dass in Abbildung 11 wegen der einfacheren Darstellbarkeit nur ein zweidimensionaler Raum gezeigt ist. Das Prinzip lässt sich aber auf beliebige höher dimensionale Räume übertragen. Sofern jedoch der Gesamtraum ausreichend niedrig dimensioniert ist, lassen sich solche Entscheidungsgrenzen auch als Unterstützung mit den tatsächlichen Datenpunkten visualisieren.

Umgekehrt lassen sich auch hier wieder visuell identifizierte Entscheidungsgrenzen auf einfache, konventionelle Entscheidungsregeln zurückführen. Weiterhin können bekannte Cluster-Zentren oder entsprechende Wahrscheinlichkeitsdichteverteilungen angezeigt werden.

Um einen möglichen räumlichen Zusammenhang zu überprüfen, könnte der Benutzer einen Teil der z. B. als "Non-Responder" markierten Bildpunkte im Parameterraum selektieren. Dies ist beispielsweise wieder durch eine Freihand-Segmentierung möglich. Diese selektierten Datenpunkte können dann, wie oben beschrieben, als Back-Mapping einem geeigneten anatomischen Bild überlagert werden, um im anatomischen Bild bzw. in der Ortsraumdarstellung die entsprechenden Bildpunkte zu markieren. Es kann dabei auch ein gleichzeitiges Back-Mapping aller Datenpunkte erfolgen, bei dem jeder Punkt entsprechend seiner Klassenzugehörigkeit farbkodiert wird.

Auch hier ist wiederum eine Datenanalyse mit Hilfe von Machine-Learning-Verfahren möglich. Vorzugsweise werden daher beispielsweise künstliche neuronale Netze eingesetzt, um aus den beobachteten Parameterkombinationen den Therapieerfolg vorherzusagen.

### Beispiel 3: Computergestützte Beurteilung von Tumorgraden bei Prostatakarzinomen

Hier ist es das Ziel, durch eine multiparametrische MRT den Tumorgrad bei Prostatakarzinomen zu bestimmen, um den Patienten so eine möglicherweise unnötige Biopsie oder gar Totalresektion zu ersparen. Bei einer abgewandelten Fragestellung könnte im Rahmen einer Verlaufskontrolle automatisch erkannt werden, wenn sich eine bislang unauffällige oder gutartige Region zu einer bösartigen Läsion entwickelt.

Die Untersuchung besteht dabei aus mehreren MR-Kontrasten, darunter üblicherweise auch eine kontrastmittelgestützte Perfusions-Dynamik.

Auch hier können wieder der Verlauf der Bildintensität jedes einzelnen Bildpunkts sowie zusätzliche Attribute, d. h. die räumlichen Koordinaten, der Akquisitionsparameter, das Prostatavolumen, Blutwerte wie PSA, als Parametervektor interpretiert werden. Werden zwölf MR-Kontraste für jeden Bildpunkt durchgeführt, ergibt sich auch hier wieder ein mindestens 12-dimensionaler Parametervektor.

Eine Dimensionsreduzierung lässt sich dabei bereits durchführen, indem für jeden einzelnen Bildpunkt Modellparameter wie ADC oder Ktrans berechnet werden. Diese Parameter sind Bestandteil etablierter parametrischer Modelle für den Signalverlauf in der Diffusionsbildgebung mit unterschiedlicher Diffusionswichtung bzw. aus der Kontrastmitteldynamik zu unterschiedlichen Zeitpunkten.

Weiterhin ist auch hier eine Reduzierung der Dimensionen mit Hilfe einer Hauptkomponentenanalyse möglich. Beispielsweise wäre mit Hilfe einer Hauptkomponentenanalyse eine Reduktion von den drei Parametern ADC, Ktrans, T2-Signalintensität auf zwei Parameter möglich.

Der mit Hilfe des Verfahrens ermittelte Grad des Tumors oder die Wahrscheinlichkeit, dass ein Bildpunkt einem klinisch signifikanten Tumor entspricht, kann als neue Parameterkarte farbkodiert dem Ortsraum bzw. dem anatomischen Bild überlagert werden, um die räumliche Zuordnung zu erleichtern. Ebenso ist auch hier natürlich ein Machine-Learning-Verfahren einsetzbar.

### Beispiel 4: Scatter-Plot-Analyse von multiparametrischen Magnetresonanzdaten

Ein Ziel ist hier die frühe Beurteilung von Therapieansprechen aufgrund von multiparametrischen Magnetresonanzmessungen zu einem oder mehreren Zeitpunkten ähnlich dem zweiten Ausführungsbeispiel.

Vor, während bzw. nach der Therapie werden auch hierbei typischerweise diverse MR-Kontraste akquiriert, z. B. jeweils zwölf Volumina. Anstatt nun wie bisher mit Hilfe von Mittelwerten für das ADC, das VE oder ähnlichen Werten für ganze Bereiche zu arbeiten, werden nun wieder im Rahmen der Erfindung der Verlauf der Bildintensität jedes einzelnen Bildpunkts sowie zusätzliche Attribute als Parametervektor interpretiert. Eine Untersuchung von insgesamt p Parametern führt dann mindestens zu einer p-dimensionalen Werteverteilung.

Auch hierbei ist wieder eine Parameterreduzierung für einzelne Bildpunkte möglich, indem bestimmte Parameterwerte miteinander verrechnet - beispielsweise Differenzen oder Verhältnisse gebildet - werden, um so die Anzahl der Werte innerhalb eines Wertetupels zu reduzieren.

Auch sind hier wieder eine Hauptkomponentenanalyse oder ähnliche Arten der Dimensionsreduzierung möglich.

Weiterhin kann auch hierbei das betrachtete Zielvolumen analysiert werden, indem Unterbereiche im Parameterraum ausgewählt werden und die in diesem Bereich liegenden Wertetupel z. B. in unterschiedlichen Farben in einem Back-Mapping-Prozess im ursprünglichen Ortsraum bzw. dem anatomischen Bild markiert werden, um so einen räumlichen Zusammenhang von bestimmten Wertebereichen des Parameterraums und dem anatomischen Bild zu erlauben.

Zusätzlich können die Daten (wie im Übrigen auch bei allen anderen Beispielen) auch in Form von Histogrammen dargestellt werden. Ein Beispiel hierfür wäre ein Histogramm, welches die Verteilung des ADC-Werts für die einzelnen Bildpunkte zeigt. Beispielsweise würde eine Änderung der Histogrammverteilung von einer monomodalen Verteilung mit einem einzelnen Peak um einen bestimmten ADC-Wert, der im Bereich eines typischen ADC-Werts von Tumorgewebe liegt, hin zu größeren und niedrigen Werten (d. h. zu einer bimodalen Verteilung), die Interpretation erlauben, dass immer mehr Bildpunkte eine Nekrose zeigen, und ebenso immer mehr Bildpunkte einen niedrigen ADC-Wert aufweisen, entsprechend dem normalen ADC-Wert von Knochenmark. Die Anzahl der Bildpunkte, die einen Wert von Tumorgewebe zeigen, geht dagegen zurück. Dies lässt dann auf einen Therapieerfolg schließen.

Eine solche Histogrammdarstellung kann automatisch, semiautomatisch oder, wenn sie einfach darstellbar ist, auch manuell ausgewertet werden, um bestimmte Grenzlinien zu ziehen und die einzelnen Wertetupel für die einzelnen Bildpunkte bestimmten Klassen zuzuordnen und diese Klassenzugehörigkeit dann wieder im Rahmen eines Back-Mappings in den Ortsraum darzustellen. Dadurch lässt sich wiederum darstellen, in welchen räumlichen Bereichen die Therapie wirkt. Auf diese Weise können also auch räumliche Parameter, wie z. B. der räumliche Schwerpunkt im anatomischen Bild, bestimmte Kontraste oder Kombinationen von Kontrasten, hinsichtlich einer klinischen Relevanz geprüft werden. Beispielsweise ist es bekannt, dass periphere Metastasen im Vergleich zu Metastasen im Körperstamm die Prognose erheblich verschlechtern. Dies ist im Rahmen des erfindungsgemäßen Verfahrens einfacher, als bisher erkennbar.

Ein großer Vorteil des erfindungsgemäßen Verfahrens ist auch, dass eine automatische Analyse der Verteilung, z. B. der Heterogenität, oder eine automatische Bestimmung der Anzahl der Moden eines Histogramms bzw. die Anzahl der Cluster eine klinische Interpretation der Daten beschleunigen und eine Quantifizierung erleichtert kann, um eine noch größere Objektivität zu schaffen.

Hier können sich besonders Machine-Learning-Verfahren anbieten. Beispielsweise kann zur Bestimmung der Moden in einem zweidimensionalen Histogramm oder in einem höher dimensionalen Raum zur Auffindung von Clustern ein Expectation-Maximization-Algorithmus im Rahmen eines Machine-Learning-Verfahrens genutzt werden. Weiterhin können wieder in einer Scatter-Plot (= Punktwolke) Entscheidungsgrenzen eingezeichnet oder automatisch gelegt werden, die vorher mit Hilfe von Machine-Learning-Algorithmen anhand von Daten anderer Patienten mit ähnlichen klinischen Fragestellungen eingelernt wurden.

In Kombination mit einer automatischen Organsegmentierung können auch z. B. jeweils unterschiedliche Kriterien pro Organ und Körperregion angewendet werden, um so die Befundung noch weiter zu strukturieren oder organspezifische Darstellungen der Parameterverteilungen zu repräsentieren.

Es wird abschließend noch einmal darauf hingewiesen, dass es sich bei den vorhergehend detailliert beschriebenen Steuervorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso ist nicht ausgeschlossen, dass als einzelne Einheiten oder Module bezeichnete und/oder dargestellte Elemente der vorliegenden Erfindung aus mehreren zusammenwirkenden Teil-Komponenten bestehen, die gegebenenfalls auch räumlich verteilt sein können.

## Patentansprüche

1. Verfahren zur Ermittlung von Ergebnisdaten (ED) auf Basis von medizinischen Messdaten (MD1, MD2, MD3, MD4, MD5) eines Untersuchungsobjekts (O), welche in verschiedenen Messungen (Ia, Ib, Id, Ic, Ie), vorzugsweise mit unterschiedlichen Messgeräten (1, 2, 3), erfasst wurden, mit folgenden Verfahrensschritten:
- Bildung eines hochdimensionalen ersten Parameterraums (PR, PR1), in welchem Messwerte der verschiedenen Messungen (Ia, Ib, Id, Ic, Ie) mit Hilfe von Wertetupeln (TP1) repräsentiert sind, wobei die Messwerte der verschiedenen Messungen (Ia, Ib, Id, Ic, Ie) anhand ihrer räumlichen Anordnung im Untersuchungsobjekt (O) und/oder anhand ihrer zeitlichen Anordnung zueinander einem Wertetupel (TP1) zugeordnet sind,
- Analyse der Wertetupel (TP1) im hochdimensionalen ersten Parameterraum (PR1) unter Nutzung zumindest einer Abbildungsfunktion (AF1) auf zumindest einen weiteren Parameterraum (PR2), welcher eine niedrigere Dimension als der erste Parameterraum (PR1) aufweist, zum Erhalt von Ergebnisdaten (ED),
- Ausgabe, vorzugsweise Visualisierung, der Ergebnisdaten (ED).

2. Verfahren nach Anspruch 1, wobei die verschiedenen Messungen gleichartige Messungen zu verschiedenen Zeitpunkten umfassen.

3. Verfahren nach Anspruch 1 oder 2, wobei die Abbildungsfunktion (AF1) zumindest eine der folgenden Funktionstypen umfasst:
- eine Abbildung in einen Farbraum,
- eine Abbildung in diskrete Klassen,
- eine Abbildung in einen Raum mit klinisch relevanten semantischen Koordinatenachsen,
- eine Abbildung in ein Koordinatensystem mit mathematischen Vorzugseigenschaften, insbesondere unter Nutzung einer Hauptachsentransformation,
- eine Integration und/oder Projektion über definierte Bereiche des Parameterraums.

4. Verfahren nach einem der vorstehenden Ansprüche, wobei zum Erhalt der Ergebnisdaten (ED) zunächst durch eine erste Abbildungsfunktion (AF1) vom ersten Parameterraum (PR1) auf einen zweiten Parameterraum (PR2) Interims-Ergebnisdaten ermittelt werden und diese Interims-Ergebnisdaten analysiert werden, wobei die Analyse unter Nutzung zumindest einer zweiten Abbildungsfunktion (AF2) auf zumindest einen dritten Parameterraum (PR3) erfolgt, welcher eine niedrigere Dimension als der zweite Parameterraum (PR2) aufweist,
wobei vorzugsweise die erste Abbildungsfunktion (AF1) und die zweite Abbildungsfunktion (AF2) unterschiedliche Funktionstypen umfassen.

5. Verfahren nach einem der vorstehenden Ansprüche, wobei die Analyse unter Berücksichtigung von Positionen der Wertetupel (TP1, TP2, TP3) in einem Parameterraum (PR, PR2) in Bezug zu einer Grenz-Hyperebene (HE1, HE2, ..., HE7) des Parameterraums (PR, PR2) erfolgt.

6. Verfahren nach einem der vorstehenden Ansprüche, wobei die Analyse eine Zuordnung der Wertetupel zu Wertetupel-Gruppen (C1, C2, ..., C8) umfasst.

7. Verfahren nach Anspruch 6, umfassend eine Analyse der Anordnung einer Wertetupel-Gruppe (C1, C2, ..., C8) im Parameterraum (PR).

8. Verfahren nach Anspruch 7, wobei die Anordnung der Wertetupel-Gruppe (C1, C2, ..., C8) unter Berücksichtigung einer kollektiven Position (MP1, MP2) der Wertetupel-Gruppe (C1, C2, ..., C8) im Parameterraum (PR) bestimmt wird.

9. Verfahren nach einem der vorstehenden Ansprüche, wobei die Analyse der Wertetupel (TP1, TP2, TP3) unter Berücksichtigung einer Position in Bezug zu zumindest einem Referenz-Wertetupel (RT) erfolgt.

10. Verfahren nach einem der vorstehenden Ansprüche, wobei die Analyse der Wertetupel eine Segmentierung (SG) umfasst.

11. Verfahren nach einem der vorstehenden Ansprüche, wobei die Analyse morphologische Operationen (MO) und/oder Filterungen (FI) umfasst.

12. Verfahren nach einem der vorstehenden Ansprüche, umfassend eine zeitliche Analyse der Veränderung von Wertetupeln im Parameterraum, vorzugsweise eine zeitliche Verschiebung (ZV) von einzelnen Wertetupeln oder der Anordnung einer Wertetupel-Gruppe (C1).

13. Verfahren nach einem der vorstehenden Ansprüche, wobei die Analyse ein maschinelles Lernverfahren umfasst.

14. Verfahren nach einem der vorstehenden Ansprüche, wobei die Visualisierung der Ergebnisdaten eine, vorzugsweise farbliche, Markierung (M1, M2, M3) von Bildpunkten und/oder Regionen in einer Ortsraumdarstellung (ORD) des Untersuchungsobjekts umfasst, wobei die Markierung in Abhängigkeit von einem Ergebnis der Analyse der Wertetupel (TP1, TP2, TP3) in dem ersten und/oder einem weiteren Parameterraum (PR, PR1, PR2, PR3) erfolgt.

15. Verfahren nach Anspruch 13 und Anspruch 14, wobei die Visualisierung der Ergebnisdaten eine Visualisierung der zeitlichen Analyse durch Farben und/oder Vektoren (ZV) umfasst.

16. Einrichtung (10) zur Ermittlung von Ergebnisdaten (ED) auf Basis von medizinischen Messdaten (MD1, MD2, MD3, MD4, MD5) eines Untersuchungsobjekts (O), welche in verschiedenen Messungen (Ia, Ib, Id, Ic, Ie), vorzugsweise mit unterschiedlichen Messgeräten (1, 2, 3), erfasst wurden,
wobei die Einrichtung (10) zur Ausführung der folgenden Verfahrensschritte ausgebildet ist:
- Bildung eines hochdimensionalen ersten Parameterraums (PR, PR1), in welchem Messwerte der verschiedenen Messungen (Ia, Ib, Id, Ic, Ie) mit Hilfe von Wertetupeln (TP1) repräsentiert sind, wobei die Messwerte der verschiedenen Messungen (Ia, Ib, Id, Ic, Ie) anhand ihrer räumlichen Anordnung im Untersuchungsobjekt (O) und/oder anhand ihrer zeitlichen Anordnung zueinander einem Wertetupel (TP1) zugeordnet sind,
- Analyse der Wertetupel (TP1) im hochdimensionalen ersten Parameterraum (PR1) unter Nutzung zumindest einer Abbildungsfunktion (AF1) auf zumindest einen weiteren Parameterraum (PR2), welcher eine niedrigere Dimension als der erste Parameterraum (PR1) aufweist, zum Erhalt von Ergebnisdaten (ED),
- Ausgabe, vorzugsweise Visualisierung, der Ergebnisdaten (ED).

17. Computerprogrammprodukt mit einem Computerprogramm, welches direkt in eine Speichereinrichtung einer Rechnereinheit (20) ladbar ist, mit Programmabschnitten, um alle Schritte des Verfahrens nach einem der Ansprüche 1 bis 15 auszuführen, wenn das Computerprogramm in der Recheneinrichtung (20) ausgeführt wird.

18. Computerlesbares Medium, auf welchem von einer Rechnereinheit (20) einlesbare und ausführbare Programmabschnitte gespeichert sind, um alle Schritte des Verfahrens nach einem der Ansprüche 1 bis 15 auszuführen, wenn die Programmabschnitte von der Rechnereinheit (20) ausgeführt werden.

## Geänderte Patentansprüche

### Geänderte Patentansprüche gemäss Regel 137(2) EPÜ.

1. Verfahren zur Ermittlung von Ergebnisdaten (ED) auf Basis von medizinischen Messdaten (MD1, MD2, MD3, MD4, MD5) eines Untersuchungsobjekts (O), welche in verschiedenen Messungen (Ia, Ib, Id, Ic, Ie), vorzugsweise mit unterschiedlichen Messgeräten (1, 2, 3), erfasst wurden, mit folgenden Verfahrensschritten:
- Bildung eines hochdimensionalen ersten Parameterraums (PR, PR1), in welchem Messwerte der verschiedenen Messungen (Ia, Ib, Id, Ic, Ie) mit Hilfe von Wertetupeln (TP1) repräsentiert sind, wobei die Messwerte der verschiedenen Messungen (Ia, Ib, Id, Ic, Ie) anhand ihrer räumlichen Anordnung im Untersuchungsobjekt (O) und/oder anhand ihrer zeitlichen Anordnung zueinander einem Wertetupel (TP1) zugeordnet sind,
- Analyse der Wertetupel (TP1) im hochdimensionalen ersten Parameterraum (PR1) unter Nutzung zumindest einer Abbildungsfunktion (AF1) auf zumindest einen weiteren Parameterraum (PR2), welcher eine niedrigere Dimension als der erste Parameterraum (PR1) aufweist, zum Erhalt von Ergebnisdaten (ED), wobei die Analyse eine Zuordnung der Wertetupel zu Wertetupel-Gruppen (C1, C2, ..., C8) mit einer Analyse der Anordnung einer Wertetupel-Gruppe (C1, C2, ..., C8) im Parameterraum (PR) umfasst,
- Ausgabe, vorzugsweise Visualisierung, der Ergebnisdaten (ED) .

2. Verfahren nach Anspruch 1, wobei die verschiedenen Messungen gleichartige Messungen zu verschiedenen Zeitpunkten umfassen.

3. Verfahren nach Anspruch 1 oder 2, wobei die Abbildungsfunktion (AF1) zumindest eine der folgenden Funktionstypen umfasst:
- eine Abbildung in einen Farbraum,
- eine Abbildung in diskrete Klassen,
- eine Abbildung in einen Raum mit klinisch relevanten semantischen Koordinatenachsen,
- eine Abbildung in ein Koordinatensystem mit mathematischen Vorzugseigenschaften.

4. Verfahren nach einem der vorstehenden Ansprüche, wobei die Abbildungsfunktion (AF1) eine Hauptachsentransformation umfasst.

5. Verfahren nach einem der vorstehenden Ansprüche, wobei die Abbildungsfunktion (AF1) eine Integration und/oder Projektion über definierte Bereiche des Parameterraums umfasst.

6. Verfahren nach einem der vorstehenden Ansprüche, wobei zum Erhalt der Ergebnisdaten (ED) zunächst durch eine erste Abbildungsfunktion (AF1) vom ersten Parameterraum (PR1) auf einen zweiten Parameterraum (PR2) Interims-Ergebnisdaten ermittelt werden und diese Interims-Ergebnisdaten analysiert werden, wobei die Analyse unter Nutzung zumindest einer zweiten Abbildungsfunktion (AF2) auf zumindest einen dritten Parameterraum (PR3) erfolgt, welcher eine niedrigere Dimension als der zweite Parameterraum (PR2) aufweist,
wobei vorzugsweise die erste Abbildungsfunktion (AF1) und die zweite Abbildungsfunktion (AF2) unterschiedliche Funktionstypen umfassen.

7. Verfahren nach einem der vorstehenden Ansprüche, wobei die Analyse unter Berücksichtigung von Positionen der Wertetupel (TP1, TP2, TP3) in einem Parameterraum (PR, PR2) in Bezug zu einer Grenz-Hyperebene (HE1, HE2, ..., HE7) des Parameterraums (PR, PR2) erfolgt.

8. Verfahren nach einem der vorstehenden Ansprüche, wobei die Anordnung der Wertetupel-Gruppe (C1, C2, ..., C8) unter Berücksichtigung einer kollektiven Position (MP1, MP2) der Wertetupel-Gruppe (C1, C2, ..., C8) im Parameterraum (PR) bestimmt wird.

9. Verfahren nach einem der vorstehenden Ansprüche, wobei die Analyse der Wertetupel (TP1, TP2, TP3) unter Berücksichtigung einer Position in Bezug zu zumindest einem Referenz-Wertetupel (RT) erfolgt.

10. Verfahren nach einem der vorstehenden Ansprüche, wobei die Analyse der Wertetupel eine Segmentierung (SG) umfasst.

11. Verfahren nach einem der vorstehenden Ansprüche, wobei die Analyse morphologische Operationen (MO) und/oder Filterungen (FI) umfasst.

12. Verfahren nach einem der vorstehenden Ansprüche, umfassend eine zeitliche Analyse der Veränderung von Wertetupeln im Parameterraum, vorzugsweise eine zeitliche Verschiebung (ZV) von einzelnen Wertetupeln oder der Anordnung einer Wertetupel-Gruppe (C1).

13. Verfahren nach einem der vorstehenden Ansprüche, wobei die Analyse ein maschinelles Lernverfahren umfasst.

14. Verfahren nach einem der vorstehenden Ansprüche, wobei die Visualisierung der Ergebnisdaten eine, vorzugsweise farbliche, Markierung (M1, M2, M3) von Bildpunkten und/oder Regionen in einer Ortsraumdarstellung (ORD) des Untersuchungsobjekts umfasst, wobei die Markierung in Abhängigkeit von einem Ergebnis der Analyse der Wertetupel (TP1, TP2, TP3) in dem ersten und/oder einem weiteren Parameterraum (PR, PR1, PR2, PR3) erfolgt.

15. Verfahren nach Anspruch 13 und Anspruch 14, wobei die Visualisierung der Ergebnisdaten eine Visualisierung der zeitlichen Analyse durch Farben und/oder Vektoren (ZV) umfasst.

16. Einrichtung (10) zur Ermittlung von Ergebnisdaten (ED) auf Basis von medizinischen Messdaten (MD1, MD2, MD3, MD4, MD5) eines Untersuchungsobjekts (O), welche in verschiedenen Messungen (Ia, Ib, Id, Ic, Ie), vorzugsweise mit unterschiedlichen Messgeräten (1, 2, 3), erfasst wurden,
wobei die Einrichtung (10) zur Ausführung der folgenden Verfahrensschritte ausgebildet ist:
- Bildung eines hochdimensionalen ersten Parameterraums (PR, PR1), in welchem Messwerte der verschiedenen Messungen (Ia, Ib, Id, Ic, Ie) mit Hilfe von Wertetupeln (TP1) repräsentiert sind, wobei die Messwerte der verschiedenen Messungen (Ia, Ib, Id, Ic, Ie) anhand ihrer räumlichen Anordnung im Untersuchungsobjekt (O) und/oder anhand ihrer zeitlichen Anordnung zueinander einem Wertetupel (TP1) zugeordnet sind,
- Analyse der Wertetupel (TP1) im hochdimensionalen ersten Parameterraum (PR1) unter Nutzung zumindest einer Abbildungsfunktion (AF1) auf zumindest einen weiteren Parameterraum (PR2), welcher eine niedrigere Dimension als der erste Parameterraum (PR1) aufweist, zum Erhalt von Ergebnisdaten (ED), wobei die Analyse eine Zuordnung der Wertetupel zu Wertetupel-Gruppen (C1, C2, ..., C8) mit einer Analyse der Anordnung einer Wertetupel-Gruppe (C1, C2, ..., C8) im Parameterraum (PR) umfasst,
- Ausgabe, vorzugsweise Visualisierung, der Ergebnisdaten (ED) .

17. Computerprogrammprodukt mit einem Computerprogramm, welches direkt in eine Speichereinrichtung einer Rechnereinheit (20) ladbar ist, mit Programmabschnitten, um alle Schritte des Verfahrens nach einem der Ansprüche 1 bis 15 auszuführen, wenn das Computerprogramm in der Recheneinrichtung (20) ausgeführt wird.

18. Computerlesbares Medium, auf welchem von einer Rechnereinheit (20) einlesbare und ausführbare Programmabschnitte gespeichert sind, um alle Schritte des Verfahrens nach einem der Ansprüche 1 bis 15 auszuführen, wenn die Programmabschnitte von der Rechnereinheit (20) ausgeführt werden.
